# EUROPÄISCHE PATENTSCHRIFT

(11) **EP 3 797 673 B1**
(45) Veröffentlichungstag und Bekanntmachung des Hinweises auf die Patenterteilung: **09.08.2023**
(21) Anmeldenummer: 20198286.5
(22) Anmeldetag: 25.09.2020
(51) Int. Cl.: A61B 1/00, A61B 8/00, A61B 90/50, A61B 8/12, A61M 25/02, A61B 34/30

(54) **HALTEVORRICHTUNG ZUM HALTEN EINES MEDIZINISCHEN INSTRUMENTS**
HOLDING DEVICE FOR HOLDING A MEDICAL INSTRUMENT
DISPOSITIF DE RETENUE PERMETTANT DE RETENIR UN INSTRUMENT MÉDICAL

(30) Priorität: 27.09.2019 DE 102019214868
(43) Veröffentlichungstag der Anmeldung: 31.03.2021
(73) Patentinhaber: Corindus, Inc., Waltham, MA 02452 (US)
(72) Erfinder: Finocchi, Rodolfo, Cambridge, MA 02138 (US); Kapoor, Ankur, Plainsboro, NJ 08536 (US); Kim, Young-Ho, West Windsor, NJ 08550 (US); Girard, Erin, Madison, CT 06443 (US); Mansi, Tommaso, Plainsboro, NJ 08536 (US)
(74) Vertreter: Horn Kleimann Waitzhofer Patentanwälte PartG mbB

(56) Entgegenhaltungen:
- WO-A1-95/24864
- WO-A1-2012/087668
- WO-A2-95/07056
- KR-B1- 101 957 344
- US-A1- 2008 249 371

## Beschreibung

Die vorliegende Erfindung betrifft eine Haltevorrichtung zum Halten eines medizinischen Instruments.

Für die genaue Untersuchung eines Untersuchungsobjekts, insbesondere bei Gefäßerkrankungen und/oder Tumorerkrankungen, sowie bei Interventionen, beispielsweise zur Gefäßaufweitung und/oder zur Platzierung einer Angioplastie in dem Untersuchungsobjekt, werden häufig langgestreckte und/oder flexible medizinische Instrumente eingesetzt. Dabei kann das Untersuchungsobjekt beispielsweise ein menschlicher und/oder tierischer Patient sein. Ferner ist das medizinische Instrument häufig als chirurgisches Instrument, beispielsweise als Katheter und/oder Führungsdraht, und/oder als diagnostisches Instrument ausgebildet, beispielsweise als Endoskop, insbesondere mit einem Ultraschallkopf für eine transösophageale Echokardiographie (engl. TEE probe), und/oder ein Bronchoskop. Während die hohe mechanische Flexibilität und/oder Verformbarkeit der medizinischen Instrumente häufig eine wichtige Grundlage für den Einsatz bei einer Intervention und/oder Untersuchung darstellt, kann hierdurch eine genaue Führung erschwert werden.

Aus dem Stand der Technik sind Führungsvorrichtungen zur Führung eines Katheters bekannt, wobei der Katheter zumindest abschnittsweise innerhalb der Führungsvorrichtung durch mehrere Antriebsräder translatorisch bewegt wird. Die Führungsvorrichtung ist dabei häufig nur für ein vorgegebenes medizinisches Instrument ausgelegt. Ferner ist eine Reichweite der translatorischen Bewegung des medizinischen Instruments mittels der Führungsvorrichtung häufig auf wenige Zentimeter begrenzt.

Des Weiteren offenbart das Dokument Loschak et al., "A 4-DOF Robot for Positioning Ultrasound Imaging Catheters", in Proc ASME Des Eng Tech Conf. 2015, 5A:V05AT08A046, eine robotische Führungsvorrichtung zur Führung eines Katheters. Dabei kann es nachteilig, insbesondere durch Reibung zwischen der Führungsvorrichtung und dem Katheter, zu einer Torsion und/oder Verformung des Katheters kommen. Hierdurch kann eine genaue Führung des Katheters als medizinisches Instrument erheblich erschwert werden.

Weiterhin sind robotergestützte Vorrichtungen zur Führung von Endoskopen bekannt, wobei die Endoskope häufig speziell für die robotergestützte Vorrichtung ausgebildet sein müssen. Ferner weisen die bekannten robotergestützten Vorrichtungen häufig einen hohen Raumbedarf auf, was gerade in einem klinischen, insbesondere chirurgischen, Umfeld nachteilig sein kann.

Die KR 10-1957344 B1 beschreibt eine Verbindungsvorrichtung, die leicht abnehmbar ist und die den Durchfluss eines Schlauches für einen Patienten mit Pneumothorax einfach kontrollieren kann. Die Verbindungsvorrichtung umfasst einen Hauptkörper, der in den Körper des Patienten eingeführt wird und der eine Öffnung zur Durchflusskontrolle aufweist, sowie einen Kupplungsabschnitt, der von dem Hauptkörper abnehmbar ist, um einen externen Schlauch fluiddynamisch mit dem Hauptkörper zu verbinden.

Der Erfindung liegt daher die Aufgabe zugrunde, eine Vorrichtung zum sicheren und flexiblen Halten eines medizinischen Instruments anzugeben.

Gemäß einer ersten Variante einer Haltevorrichtung zum Halten eines medizinischen Instruments wird diese Aufgabe durch die Merkmale des Anspruchs 1 erfindungsgemäß gelöst. Vorteilhafte Ausführungsformen mit zweckmäßigen Weiterbildungen sind Gegenstand der Unteransprüche. Die Aufgabe kann ferner durch weitere Ausführungsformen der Erfindung gelöst werden, welche in der Beschreibung aufgeführt sind.

Danach ist vorgesehen, dass die Haltevorrichtung ein erstes Aufnahmeelement, ein zweites Aufnahmeelement und zumindest drei Blendenelemente umfasst. Dabei sind die zumindest drei Blendenelemente innerhalb einer Blendenschicht zwischen dem ersten und dem zweiten Aufnahmeelement um eine gemeinsame Rotationsachse des ersten und des zweiten Aufnahmeelements herum angeordnet. Dabei verläuft die gemeinsame Rotationsachse im Wesentlichen senkrecht zur Blendenschicht. Ferner weisen das erste und das zweite Aufnahmeelement jeweils eine Öffnung zur Aufnahme des medizinischen Instruments auf, insbesondere zumindest an der gemeinsamen Rotationsachse. Des Weiteren sind das erste und das zweite Aufnahmeelement um die gemeinsame Rotationsachse herum relativ zueinander beweglich. Zudem umfassen die zumindest drei Blendenelemente jeweils zumindest ein erstes Kopplungselement. Dabei umfasst das erste und/oder das zweite Aufnahmeelement zu jedem der zumindest drei Blendenelemente jeweils zumindest ein zweites Kopplungselement. Ferner ist jedes der zumindest drei Blendenelemente innerhalb der Blendenschicht, in der diese angeordnet sind, durch mechanische Kopplung zwischen dem jeweiligen zumindest einen ersten Kopplungselement des Blendenelements und dem zumindest einen zweiten Kopplungselement zwangsgeführt. Des Weiteren erfolgt bei einer Bewegung des ersten Aufnahmeelements relativ zum zweiten Aufnahmeelement um die gemeinsame Rotationsachse herum eine durch das erste und das zweite Aufnahmeelement derart zwangsgeführte Bewegung der zumindest drei Blendenelemente innerhalb der Blendenschicht hin zur Öffnung zur Aufnahme des medizinischen Instruments, dass die zumindest drei Blendenelemente ein in der Öffnung des erste und des zweiten Aufnahmeelement angeordnetes medizinisches Instrument halten.

Dabei kann das medizinische Instrument ein chirurgisches Instrument, beispielsweise einen Katheter und/oder einen Führungsdraht, und/oder ein diagnostisches Instrument umfassen, beispielsweise ein Endoskop, insbesondere mit einem Ultraschallkopf für eine transösophageale Echokardiographie (engl. TEE probe), und/oder ein Bronchoskop und/oder ein Laparoskop.

Insbesondere kann das medizinische Instrument langgestreckt und/oder mechanisch verformbar sein.

Das erste und/oder das zweite Aufnahmeelement kann vorteilhafterweise scheibenförmig ausgebildet sein. Dabei kann das erste und/oder das zweite Aufnahmeelement eine Zentralebene aufweisen. Dabei kann eine räumliche Ausdehnung des ersten und/oder des zweiten Aufnahmeelements entlang der jeweiligen Zentralebene vorzugsweise größer gegenüber einer räumlichen Ausdehnung des ersten und/oder des zweiten Aufnahmeelements senkrecht zur jeweiligen Zentralebene sein. Zudem kann das erste und/oder das zweite Aufnahmeelement einen Rand aufweisen, wobei dieser weiterhin eine Erhebung aufweisen kann. Hierdurch kann vorteilhafterweise ermöglicht werden, dass das erste und/oder das zweite Aufnahmeelement das jeweils andere Aufnahmeelement zumindest teilweise umschließt.

Ferner kann das erste und/oder das zweite Aufnahmeelement vorteilhafterweise zumindest teilweise eine mechanisch stabile, insbesondere nicht verformbare, Form aufweisen. Ferner kann die gemeinsame Rotationsachse des ersten und/oder des zweiten Aufnahmeelements jeweils eine Symmetrieachse des ersten und/oder des zweiten Aufnahmeelements bilden.

Jedes der zumindest drei Blendenelemente kann vorteilhafterweise jeweils zwei Kontaktflächen zur, insbesondere direkten, Anordnung an jeweils einer Kontaktfläche von zumindest zwei benachbarten Blendenelementen aufweisen. Hierdurch kann vorteilhafterweise eine Beweglichkeit der zumindest drei Blendenelemente bei gleichzeitiger Wahrung der Stabilität ermöglicht werden. Dabei können die zumindest zwei Kontaktflächen von jedem der zumindest drei Blendenelemente vorzugsweise eine reibungsarme Oberfläche aufweisen.

Des Weiteren kann die Blendenschicht vorteilhafterweise einen, insbesondere scheibenförmigen, räumlichen Bereich zwischen dem ersten und dem zweiten Aufnahmeelement umfassen. Insbesondere kann die Blendenschicht durch die räumliche Ausdehnung der zumindest drei Blendenelemente zwischen dem ersten und dem zweiten Aufnahmeelement gebildet werden. Dabei können die zumindest drei Blendenelemente innerhalb der Blendenschicht zwischen dem ersten und dem zweiten Aufnahmeelement um die gemeinsame Rotationsachse herum, insbesondere ringförmig, angeordnet sein. Beispielsweise können die zumindest drei Blendenelemente entlang eines gemeinsamen Kreises um die gemeinsame Rotationsachse herum innerhalb der Blendenschicht angeordnet sein.

Ferner kann das erste und/oder das zweite Aufnahmeelement, insbesondere eine Erhebung am Rand des ersten und/oder des zweiten Aufnahmeelements, vorteilhafterweise die Blendenschicht zumindest teilweise umschließen. Hierdurch kann ein Schutz, insbesondere eine Abdichtung, vor einer äußeren mechanischen und/oder chemischen Einwirkung auf die zumindest drei Blendenelemente sichergestellt werden.

Vorteilhafterweise verläuft die gemeinsame Rotationsachse des ersten und des zweiten Aufnahmeelements im Wesentlichen senkrecht, insbesondere nicht parallel, zur Blendenschicht.

Die Öffnung zur Aufnahme des medizinischen Instruments ist vorteilhafterweise derart ausgebildet, dass das medizinische Instrument vollständig und frei beweglich entlang zumindest einer Raumrichtung in die Öffnung einführbar ist. Dabei kann ein maximaler Durchmesser des medizinischen Instruments, das in die Öffnung des ersten und des zweiten Aufnahmeelements einführbar ist, durch eine räumliche Ausdehnung der Öffnung des ersten und des zweiten Aufnahmeelements begrenzt sein.

Vorteilhafterweise sind das erste und das zweite Aufnahmeelement um die gemeinsame Rotationsachse herum relativ zueinander beweglich. Dabei kann das erste und/oder das zweite Aufnahmeelement um die gemeinsame Rotationsachse herum beweglich sein. Ferner können das erste und das zweite Aufnahmeelement rotationsbeweglich relativ zueinander sein. Des Weiteren kann das erste und/oder das zweite Aufnahmeelement aus der Anordnung herausnehmbar und/oder in die Anordnung einbringbar sein.

Vorteilhafterweise können die zumindest drei Blendenelemente zwei erste Kopplungselemente aufweisen. Ferner können vorzugsweise das erste und das zweite Aufnahmeelement jeweils ein zweites Kopplungselement zu jedem der zumindest drei Blendenelemente aufweisen. Hierdurch kann jedes der zumindest drei Blendenelemente mechanisch an das erste und das zweite Aufnahmeelement mittels der jeweils zwei zweiten Kopplungselemente gekoppelt sein. Dabei kann ferner jeweils eines der zwei zweiten Kopplungselemente zu jedem der zumindest drei Blendenelemente zur Positionierung ausgebildet sein.

Vorteilhafterweise kann durch Bewegung des ersten Aufnahmeelements relativ zum zweiten Aufnahmeelements um die gemeinsame Rotationsachse herum eine Veränderung der Anordnung der zweiten Kopplungselemente des ersten und/oder des zweiten Aufnahmeelements gegenüber der Anordnung der zumindest drei Blendenelemente erfolgen. Sofern das erste und das zweite Aufnahmeelement zu jedem der zumindest drei Blendenelemente jeweils ein zweites Kopplungselement aufweisen, kann durch Bewegung des ersten Aufnahmeelements relativ zum zweiten Aufnahmeelement eine Veränderung der Anordnungen der zweiten Kopplungselemente des ersten und des zweiten Aufnahmeelements zueinander erfolgen. Durch die mechanische Kopplung zwischen den ersten Kopplungselementen der zumindest drei Blendenelemente und den zweiten Kopplungselementen kann hierdurch eine zwangsgeführte Bewegung der Blendenelemente innerhalb der Blendenschicht ermöglicht werden.

Vorteilhafterweise sind die zweiten Kopplungselemente des ersten und/oder des zweiten Aufnahmeelements derart ausgebildet, dass die zumindest drei Blendenelemente hin zur Öffnung zur Aufnahme des medizinischen Instruments zwangsgeführt bewegbar, insbesondere rotierbar und/oder verschiebbar, sind. Ferner kann zumindest eins der zweiten Kopplungselemente des ersten und/oder des zweiten Aufnahmeelements kreisförmig und/oder gekrümmt und/oder geradlinig ausgebildet sein.

Vorteilhafterweise können die zumindest drei Blendenelemente durch die mechanische Kopplung zwischen dem zumindest einen ersten Kopplungselement und dem jeweiligen zumindest einen zweiten Kopplungselement innerhalb der Blendenschicht, insbesondere innerhalb eines vorgegebenen Radius von der gemeinsamen Rotationsachse, beweglich gehalten werden.

Dabei können vorteilhafterweise alle medizinischen Instrumente, welche in die Öffnung des ersten und des zweiten Aufnahmeelements einführbar sind, von den zumindest drei Blendenelementen gehalten werden. Bei der Bewegung des ersten Aufnahmeelements relativ zum zweiten Aufnahmeelement um die gemeinsame Rotationsachse herum, kann ein Endpunkt der Bewegung durch einen mechanischen Widerstand beim Auftreffen der zumindest drei Blendenelemente an dem in der Öffnung des ersten und des zweiten Aufnahmeelements angeordneten medizinischen Instruments vorgegeben sein. Vorteilhafterweise kann das medizinische Instrument durch die zumindest drei Blendenelemente nach einer Bewegung des ersten Aufnahmeelements relativ zum zweiten Aufnahmeelement um die gemeinsame Rotationsachse herum bis zum Endpunkt der Bewegung gehalten werden. Hierbei kann eine Bewegung des ersten Aufnahmeelements relativ zum zweiten Aufnahmeelement entlang einer ersten Bewegungsrichtung derart, dass die zumindest drei Blendenelemente hin zur Öffnung zwangsgeführt bewegt werden, als Schließen der Haltevorrichtung bezeichnet werden. Analog hierzu, kann eine Bewegung des ersten Aufnahmeelements relativ zum zweiten Aufnahmeelement entlang einer zweiten Bewegungsrichtung derart, dass die zumindest drei Blendenelemente weg von der Öffnung zwangsgeführt bewegt werden, als Öffnen der Haltevorrichtung bezeichnet werden.

Dabei kann das Halten des medizinischen Instruments durch die zumindest drei Blendenelemente vorteilhafterweise mittels einer mechanischen Kopplung zwischen dem medizinischen Instrument und den zumindest drei Blendenelementen ermöglicht werden. Vorteilhafterweise kann das medizinische Instrument durch die zumindest drei Blendenelemente gegenüber der Haltevorrichtung, insbesondere nicht verschiebbar und/oder rotationsstabil, in Position gehalten werden. Dabei können die zumindest drei Blendenelemente zum Halten des medizinischen Instruments jeweils einen Druck auf das medizinische Instrument ausüben.

In einer weiteren vorteilhaften Ausführungsform der vorgeschlagenen Haltevorrichtung kann das zumindest eine erste Kopplungselement des jeweiligen Blendenelements als längliche Führung ausgebildet sein, in der jeweils zumindest ein zweites Kopplungselement des ersten und/oder des zweiten Aufnahmeelements aufgenommen ist. Dabei kann das jeweils zumindest eine zweite Kopplungselement des ersten und/oder des zweiten Aufnahmeelements derart ausgebildet sein, dass es in eine längliche Führung des jeweiligen Blendenelements aufnehmbar ist. Hierdurch kann vorteilhafterweise eine mechanische Kopplung zwischen dem jeweiligen zumindest einen zweiten Kopplungselement des ersten und/oder zweiten Aufnahmeelements und der länglichen Führung des jeweiligen Blendenelements ermöglicht werden. Vorteilhafterweise ist das jeweilige Blendenelement entlang eines Führungspfades innerhalb der länglichen Führung beweglich, beispielsweise verschiebbar. Ferner kann jeweils eine Rotationsachse zu jedem der zumindest drei Blendenelemente durch Aufnahme des zumindest einen zweiten Kopplungselements innerhalb der länglichen Führung des jeweiligen Blendenelements vorgebbar sein. Dabei kann jedes der zumindest drei Blendenelemente innerhalb der Blendenschicht vorteilhafterweise um die vorgegebene Rotationsachse rotierbar sein. Zudem kann zumindest eines der ersten Kopplungselemente des jeweiligen Blendenelements dazu ausgebildet sein, das aufgenommene zweite Kopplungselement zumindest teilweise, insbesondere vollständig, zu umschließen.

In einer weiteren vorteilhaften Ausführungsform der vorgeschlagenen Haltevorrichtung kann das zumindest eine zweite Kopplungselement des ersten und/oder des zweiten Aufnahmeelements als längliche Führung ausgebildet sein, in der das jeweils zumindest eine erste Kopplungselement des jeweiligen Blendenelements aufgenommen ist. Dabei kann das zumindest eine erste Kopplungselement von jedem der zumindest drei Blendenelemente derart ausgebildet sein, dass es in jeweils eine längliche Führung des ersten und/oder des zweiten Aufnahmeelements aufnehmbar ist. Hierdurch kann vorteilhafterweise eine mechanische Kopplung zwischen dem jeweiligen zumindest einen ersten Kopplungselement des Blendenelements und der länglichen Führung ermöglicht werden. Vorteilhafterweise ist das zumindest eine erste Kopplungselement des Blendenelements entlang eines Führungspfades innerhalb der länglichen Führung des ersten und/oder des zweiten Aufnahmeelements beweglich, beispielsweise verschiebbar. Ferner kann jeweils eine Rotationsachse zu jedem der zumindest drei Blendenelemente durch Aufnahme des zumindest einen ersten Kopplungselements innerhalb der länglichen Führung des ersten und/oder des zweiten Aufnahmeelements vorgebbar sein. Dabei kann jedes der zumindest drei Blendenelemente innerhalb der Blendenschicht vorteilhafterweise um die vorgegebene Rotationsachse rotierbar sein. Zudem kann zumindest eines der zweiten Kopplungselemente des ersten und/oder des zweiten Aufnahmeelements dazu ausgebildet sein, das aufgenommene erste Kopplungselement zumindest teilweise, insbesondere vollständig, zu umschließen.

In einer weiteren vorteilhaften Ausführungsform der vorgeschlagenen Haltevorrichtung können die zumindest drei Blendenelemente innerhalb der Blendenschicht zumindest teilweise gegenseitig überlappend angeordnet sein. Hierdurch kann eine besonders platzsparende Anordnung der zumindest drei Blendenelemente innerhalb der Blendenschicht ermöglicht werden. Ferner kann durch die zumindest teilweise überlappende Anordnung der zumindest drei Blendenelemente die mechanische Kopplung zwischen dem medizinischen Instrument und den zumindest drei Blendenelementen, insbesondere entlang einer Längsrichtung des medizinischen Instruments, verbessert werden. Des Weiteren kann eine höhere Stabilität der Anordnung der zumindest drei Blendenelemente innerhalb der Blendenschicht gegenüber einer Kraftwirkung durch das gehaltene medizinische Instrument bei einer zumindest teilweise überlappenden Anordnung der zumindest drei Blendenelemente ermöglicht werden.

In einer weiteren vorteilhaften Ausführungsform der vorgeschlagenen Haltevorrichtung kann das jeweils zumindest eine erste Kopplungselement des jeweiligen Blendenelements und/oder das zumindest eine zweite Kopplungselement des ersten und/oder des zweiten Aufnahmeelements als Erhebung und/oder Aussparung des jeweiligen Blendenelements ausgebildet sein. Dabei kann das zumindest eine erste Kopplungselement vorteilhafterweise als stiftförmige und/oder halbkugelförmige und/oder zylindrische Erhebung des jeweiligen Blendenelements ausgebildet sein. Ferner kann das zumindest eine zweite Kopplungselement als stiftförmige und/oder halbkugelförmige und/oder zylindrische Erhebung des ersten und/oder des zweiten Aufnahmeelements ausgebildet sein.

Ferner kann das zumindest eine erste Kopplungselement vorzugsweise als zylindrische und/oder halbkugelförmige Aussparung des jeweiligen Blendenelements ausgebildet sein. Des Weiteren kann das zumindest eine zweite Kopplungselement als zylindrische und/oder halbkugelförmige Aussparung des ersten und/oder des zweiten Aufnahmeelements ausgebildet sein.

Zudem kann das zumindest eine erste Kopplungselement derart als Erhebung und/oder Aussparung des jeweiligen Blendenelements ausgebildet sein, dass eine, insbesondere äußere, Formgebung des jeweiligen Blendenelements durch das zumindest eine erste Kopplungselement gebildet wird. Beispielsweise kann das zumindest eine erste Kopplungselement als eine Erhebung und/oder Aussparung an einer Seitenfläche und/oder Kante und/oder Ecke des jeweiligen Blendenelements ausgebildet sein. Zudem kann das zumindest eine zweite Kopplungselement derart als Erhebung und/oder Aussparung des ersten und/oder des zweiten Aufnahmeelements ausgebildet sein, dass eine Formgebung des ersten und/oder des zweiten Aufnahmeelements durch das zumindest eine zweite Kopplungselement gebildet wird. Beispielsweise kann das zumindest eine zweite Kopplungselement als eine Erhebung und/oder Aussparung an einer Seitenfläche und/oder Kante und/oder Ecke des ersten und/oder des zweiten Aufnahmeelements ausgebildet sein. Sofern das erste und/oder das zweite Aufnahmeelement einen Rand und/oder eine Erhebung aufweist, kann das jeweils zumindest eine zweite Kopplungselement vorteilhafterweise an dem Rand und/oder der Erhebung angeordnet sein. Hierdurch kann vorteilhafterweise eine Kontaktfläche zwischen dem zumindest einen ersten Kopplungselement des jeweiligen Blendenelements und dem korrespondierenden zumindest einen zweiten Kopplungselement des ersten und/oder des zweiten Aufnahmeelements zur gegenseitigen mechanischen Kopplung maximiert werden.

Die zumindest drei Blendenelemente weisen ein gegenüber dem ersten und/oder dem zweiten Aufnahmeelement weicheres Material auf. Dabei kann jedes der zumindest drei Blendenelemente zumindest eine Grifffläche umfassen, die bei geschlossener Haltevorrichtung das medizinische Instrument berührt. Vorteilhafterweise kann die Grifffläche ein rutschhemmendes Material und/oder anhaftendes Material aufweisen. Hierdurch kann ein besonders sicheres Halten des medizinischen Instruments durch die zumindest drei Blendenelemente ermöglicht werden.

Ferner können die zumindest drei Blendenelemente zumindest teilweise aus einem gegenüber dem ersten und/oder dem zweiten Aufnahmeelement weicheren Material, beispielsweise Viskoschaum und/oder Gummi, bestehen. Hierdurch kann ein Anschmiegen der zumindest drei Blendenelemente an das medizinische Instrument in geschlossenem Zustand der Haltevorrichtung ermöglicht werden. Hierdurch kann eine Maximierung der Grifffläche zwischen den zumindest drei Blendenelementen und dem medizinischen Instrument erreicht werden. Ferner kann eine besonders gute Anpassung der zumindest drei Blendenelemente an, insbesondere ungleichmäßig, geformte medizinische Instrumente ermöglicht werden. Hierdurch kann ein besonders flexibler Einsatz der Haltevorrichtung für viele verschieden geformte medizinische Instrumente ermöglicht werden.

In einer weiteren vorteilhaften Ausführungsform der vorgeschlagenen Haltevorrichtung können die zumindest drei Blendenelemente gleichförmig um die gemeinsame Rotationsachse herum angeordnet sein. Hierdurch kann ein besonders gleichmäßiges Halten des medizinischen Instruments durch die zumindest drei Blendenelemente ermöglicht werden. Ferner kann ein Verkanten der zumindest drei Blendenelemente bei der zwangsgeführten Bewegung innerhalb der Blendenschicht durch eine gleichmäßige Anordnung um die gemeinsame Rotationsachse herum verhindert werden. Des Weiteren kann bei einer gleichmäßigen Anordnung der zumindest drei Blendenelemente um die gemeinsame Rotationsachse herum erreicht werden, dass das medizinische Instrument an der gemeinsamen Rotationsachse, insbesondere gegenüber den zumindest drei Blendenelementen zentriert, gehalten wird.

In einer weiteren vorteilhaften Ausführungsform der vorgeschlagenen Haltevorrichtung können die zumindest drei Blendenelemente entlang der Blendenschicht eine dreieckige und/oder kreissegmentförmige Form aufweisen. Hierdurch kann eine Anordnung der zumindest drei Blendenelemente nebeneinander, insbesondere ohne gegenseitige Überlappung, innerhalb der Blendenschicht ermöglicht werden. Ferner kann durch eine dreieckige und/oder kreissegmentförmige Form der zumindest drei Blendenelemente entlang der Blendenschicht eine gegenseitige Führung der Blendenelemente bei einer Bewegung innerhalb der Blendenschicht ermöglicht werden. Dabei können vorzugsweise benachbarte Blendenelemente jeweils aneinander entlanggleiten.

In einer weiteren vorteilhaften Ausführungsform der vorgeschlagenen Haltevorrichtung kann die Öffnung zur Aufnahme des medizinischen Instruments des ersten und/oder des zweiten Aufnahmeelements als Loch und/oder Schlitz ausgebildet sein. Bei einer Ausführung der Öffnung zur Aufnahme des medizinischen Instruments als Loch kann das medizinische Instrument vorteilhafterweise zumindest entlang der gemeinsamen Rotationsachse einführbar sein. Hierbei kann ein Ende des medizinischen Instruments in die Öffnung eingeführt werden, wobei das medizinische Instrument zumindest bis zu einer vorbestimmten Position innerhalb der Öffnung verschiebbar ist.

Bei einer Ausführung der Öffnung zur Aufnahme des medizinischen Instruments als Schlitz, kann die Haltevorrichtung vorteilhafterweise auf das medizinische Instrument aufsteckbar sein. Ferner kann das medizinische Instrument durch den Schlitz seitlich in die Haltevorrichtung einbringbar sein. Hierdurch kann vorteilhafterweise ein Anbringen der Haltevorrichtung an dem medizinischen Instrument auch dann ermöglicht werden, wenn alle Enden des medizinischen Instruments befestigt sind.

Ferner kann durch eine Bewegung des ersten Aufnahmeelements relativ zum zweiten Aufnahmeelement um die gemeinsame Rotationsachse herum, eine Anordnung der beiden als schlitz ausgebildeten Öffnungen derart veränderbar sein, dass ein in der Öffnung angeordnetes medizinisches Instrument nicht seitlich aus der Haltevorrichtung herausnehmbar ist. Hierdurch kann eine besonders sichere Aufnahme des medizinischen Instruments innerhalb der Öffnung des ersten und des zweiten Aufnahmeelements ermöglicht werden.

In einer weiteren vorteilhaften Ausführungsform der vorgeschlagenen Haltevorrichtung kann das erste und/oder das zweite Aufnahmeelement radförmig ausgebildet sein. Dabei kann das erste und/oder das zweite Aufnahmeelement vorteilhafterweise entlang der jeweiligen Zentralebene rund und scheibenförmig ausgebildet sein, insbesondere um die gemeinsame Rotationsachse herum. Vorteilhafterweise sind das erste und/oder das zweite Aufnahmeelement um die gemeinsame Rotationsachse herum rotationsbeweglich zueinander. Bei einer radförmigen Ausführung des ersten und/oder des zweiten Aufnahmeelements kann eine besonders einfache Rotationsbewegung des ersten Aufnahmeelements relativ zum zweiten Aufnahmeelement um die gemeinsame Rotationsachse herum ermöglicht werden. Ferner kann eine radförmige Ausbildung des ersten und/oder des zweiten Aufnahmeelements besonders platzsparend sein.

In einer weiteren vorteilhaften Ausführungsform der vorgeschlagenen Haltevorrichtung kann das erste und/oder das zweite Aufnahmeelement als Zahnrad und/oder Riemenrad ausgebildet sein. Hierdurch kann vorteilhafterweise eine angetriebene Bewegung des ersten und/oder des zweiten Aufnahmeelements ermöglicht werden. Dabei bezeichnet eine Ausbildung als Zahnrad vorteilhafterweise eine Anordnung von zahnförmigen, insbesondere dreieckigen, Erhebungen entlang eines Umfangs des radförmigen ersten und/oder zweiten Aufnahmeelements. Ferner kann das erste und/oder das zweite Aufnahmeelement vorteilhafterweise eine Aussparung entlang des Umfangs aufweisen, wobei ein Riemen zum Antrieb der Bewegung des ersten und/oder des zweiten Aufnahmeelements innerhalb der Aussparung angelegt werden kann. Bei einer Ausbildung des ersten und/oder des zweiten Aufnahmeelements als Zahnrad und/oder Riemenrad kann eine verbesserte mechanische Kopplung, beispielsweise durch Reibung, an ein Antriebselement ermöglicht werden.

In einer weiteren vorteilhaften Ausführungsform der vorgeschlagenen Haltevorrichtung kann die Haltevorrichtung weiterhin ein motorisiertes Antriebselement umfassen, das zur Bewegung des ersten Aufnahmeelements relativ zum zweiten Aufnahmeelement um die gemeinsame Rotationsachse herum ausgebildet ist. Dabei kann das motorisierte Antriebselement einen Motor, insbesondere einen Elektromotor, umfassen. Ferner kann das motorisierte Antriebselement vorteilhafterweise ein Übertragungselement umfassen, welches den Motor mechanisch an das erste und/oder das zweite Aufnahmeelement koppelt. Dabei kann das Übertragungselement beispielsweise als Riemen und/oder Zahnrad und/oder Getriebe ausgebildet sein. Vorteilhafterweise kann durch das motorisierte Antriebselement zur Bewegung des ersten Aufnahmeelements relativ zum zweiten Aufnahmeelements eine besonders präzise und/oder ferngesteuerte Bewegung ermöglicht werden. Insbesondere kann die Haltevorrichtung zum Halten des medizinischen Instruments mittels des motorisierten Antriebselements besonders einfach und/oder wiederholt geöffnet und geschlossen werden.

In einer weiteren vorteilhaften Ausführungsform der vorgeschlagenen Haltevorrichtung kann die Haltevorrichtung weiterhin ein Feststellelement umfassen, das dazu ausgebildet ist, das erste Aufnahmeelement gegenüber dem zweiten Aufnahmeelement zu arretieren. Das medizinische Instrument wird durch die zumindest drei Blendenelemente nur solange festgehalten, wie das erste Aufnahmeelement und das zweite Aufnahmeelement im geschlossenen Zustand der Haltevorrichtung relativ zueinander in Ruhe verharren. Ein Feststellelement, das dazu ausgebildet ist, das erste Aufnahmeelement gegenüber dem zweiten Aufnahmeelement, insbesondere positionsfest, zu arretieren, kann daher vorteilhaft für ein sicheres Halten des medizinischen Instruments sein. Insbesondere kann das erste Aufnahmeelement gegenüber dem zweiten Aufnahmeelement im geschlossenen Zustand der Haltevorrichtung durch das Feststellelement arretiert werden.

Dabei kann das Feststellelement insbesondere als Verriegelung, beispielsweise ein Hebel und/oder eine Steckverbindung zwischen dem ersten und dem zweiten Aufnahmeelement, und/oder als Teil des motorisierten Antriebselements ausgebildet sein. Des Weiteren kann das Feststellelement zumindest einen Magneten aufweisen. Ferner kann das Feststellelement auf das erste und/oder das zweite Aufnahmeelement aufsteckbar und/oder in das erste und/oder in das zweite Aufnahmeelement einbringbar sein. Hierdurch kann das erste mit dem zweiten Aufnahmeelement, insbesondere reversibel, mechanisch starr verbunden werden, wobei eine Bewegung des ersten gegenüber dem zweiten Aufnahmeelement durch das Feststellelement verhindert werden kann. Ferner kann das erste gegenüber dem zweiten Aufnahmeelement abhängig von einer Ausrichtung und/oder Orientierung arretierbar sein.

Ferner kann die Arretierung des ersten Aufnahmeelements gegenüber dem zweiten Aufnahmeelement mittels des Feststellelements vorzugsweise lösbar sein. Insbesondere kann durch das Lösen der Arretierung des ersten Aufnahmeelements gegenüber dem zweiten Aufnahmeelement ein Öffnen der Haltevorrichtung ermöglicht werden.

In einer weiteren vorteilhaften Ausführungsform der vorgeschlagenen Haltevorrichtung kann die Haltevorrichtung weiterhin ein Blendenfeststellelement umfassen, das dazu ausgebildet ist, die zumindest drei Blendenelemente zu arretieren. Dabei kann das Blendenfeststellelement vorteilhafterweise innerhalb der Blendenschicht und/oder um die Blendenschicht herum angeordnet sein. Vorteilhafterweise kann zumindest eines der Blendenelemente durch das Blendenfeststellelement positionsfest arretierbar sein. Insbesondere bei einer Anordnung der zumindest drei Blendenelemente ohne gegenseitige Überlappung innerhalb der Blendenschicht kann eine Arretierung eines einzelnen Blendenelements zur Arretierung aller Blendenelemente ausreichen. Dies kann insbesondere durch gegenseitige Reibung der Blendenelemente aneinander ermöglicht werden. Ferner kann das Blendenfeststellelement dazu ausgebildet sein, das zumindest eine erste Kopplungselement zumindest eines Blendenelements an das korrespondierende zumindest eine zweite Kopplungselement des ersten und/oder des zweiten Aufnahmeelements starr, insbesondere bewegungsfest, mechanisch zu koppeln.

Ferner kann das Blendenfeststellelement dazu ausgebildet sein, die Anordnung der zumindest drei Blendenelemente, insbesondere in geschlossenem Zustand der Haltevorrichtung, zu arretieren. Dabei kann das Blendenfeststellelement beispielsweise als umlaufendes Band und/oder Ring und/oder Hebel und/oder Schelle ausgebildet sein. Vorteilhafterweise kann die Haltevorrichtung durch Lösen der Arretierung der zumindest drei Blendenelemente geöffnet werden. Bei einer Anordnung des Blendenfeststellelements innerhalb der Blendenschicht kann eine besonders platzsparende Ausführung ermöglicht werden.

Vorteilhafterweise kann durch eine Arretierung der zumindest drei Blendenelemente durch das Blendenfeststellelement weiterhin eine Bewegung des ersten und/oder des zweiten Aufnahmeelements ermöglicht werden. Beispielsweise kann das erste und/oder das zweite Aufnahmeelement nach Schließen der Haltevorrichtung und Arretierung der zumindest drei Blendenelemente durch das Blendenfeststellelement entfernbar sein. Hierdurch kann das erste und/oder das zweite Aufnahmeelement vorzugsweise bei einer weiteren Haltevorrichtung einsetzbar sein.

Ferner kann es vorteilhaft sein, wenn das Blendenfeststellelement und/oder eines der zumindest drei Blendenelemente weiterhin ein Befestigungselement zur Befestigung an einem medizinischen Gerät und/oder einer Patientenlagerungsvorrichtung umfasst. Hierdurch kann das medizinische Instrument vorteilhafterweise mittels der Haltevorrichtung an dem medizinischen Gerät und/oder der Patientenlagerungsvorrichtung gehalten werden.

In einer weiteren vorteilhaften Ausführungsform der vorgeschlagenen Haltevorrichtung kann das erste Aufnahmeelement zu jedem der zumindest drei Blendenelemente jeweils ein zweites Kopplungselement umfassen, das als längliche Führung ausgespart ist umfassen, die als Aussparung ausgebildet ist und geradlinig verläuft. Ferner kann das zweite Aufnahmeelement zu jedem der zumindest drei Blendenelemente jeweils ein weiteres zweites Kopplungselement umfassen, das als längliche Führung ausgespart ist und gekrümmt verläuft. Dabei können das erste und das zweite Aufnahmeelement radförmig ausgebildet sein. Des Weiteren kann das erste und/oder das zweite Aufnahmeelement als Zahnrad ausgebildet sein. Dabei kann die Öffnung zur Aufnahme des medizinischen Instruments des erste und des zweiten Aufnahmeelements als Schlitz ausgebildet sein und von der gemeinsamen Rotationsachse begrenzt werden. Ferner können die zumindest drei Blendenelemente eine dreieckige Form aufweisen. Dabei kann jedes der zumindest drei Blendenelemente jeweils zwei erste Kopplungselemente aufweisen, die als stiftförmige Erhebungen ausgebildet sind. Zudem kann jeweils ein erstes der zwei ersten Kopplungselemente von jedem der zumindest drei Blendenelemente auf einer dem ersten Aufnahmeelement zugewandten Seite des Blendenelements angeordnet sein. Dabei kann jeweils ein zweites der zwei ersten Kopplungselemente von jedem der zumindest drei Blendenelemente auf einer dem zweiten Aufnahmeelement zugewandten Seite des Blendenelements angeordnet sein. Ferner können die zwei ersten Kopplungselemente von jedem der zumindest drei Blendenelemente nicht auf einer zur gemeinsamen Rotationsachse parallelen Raumachse liegen.

Hierdurch kann eine besonders platzsparende Ausführung der vorgeschlagenen Haltevorrichtung ermöglicht werden.

Die Erfindung betrifft in einem weiteren Aspekt eine Bewegungsvorrichtung zur Bewegung eines medizinischen Instruments umfassend eine Haltevorrichtung, ein Übertragungselement und ein Verbindungselement. Dabei kann die Haltevorrichtung vorteilhafterweise zum Halten des medizinischen Instruments ausgebildet. Ferner kann das Übertragungselement eine Bewegung zwischen zumindest einem Teil der Haltevorrichtung und dem Verbindungselement, insbesondere bidirektional, übertragen. Des Weiteren kann das Verbindungselement zur Haltevorrichtung beabstandet angeordnet sein.

Dabei kann die Haltevorrichtung insbesondere eine vorstehend beschriebene vorgeschlagene Haltevorrichtung sein. Ferner kann das Übertragungselement vorteilhafterweise einen Riemen und/oder ein Zahnrad und/oder ein Getriebe aufweisen. Hierdurch kann eine besonders präzise und direkte Übertragung einer Bewegung zwischen zumindest einem Teil der Haltevorrichtung und dem Verbindungselement ermöglicht werden. Vorteilhafterweise kann eine Bewegung zwischen dem ersten und/oder dem zweiten Aufnahmeelement und/oder den zumindest drei Blendenelementen der Haltevorrichtung und dem Verbindungselement, insbesondere bidirektional, übertragen werden. Ferner kann eine Bewegung der Haltevorrichtung als Ganzes, insbesondere in geschlossenem Zustand mit dem darin angeordneten medizinischen Instrument, zwischen der Haltevorrichtung und dem Verbindungselement durch das Übertragungselement übertragbar sein. Dabei kann die Bewegung vorteilhafterweise eine Rotationsbewegung der Haltevorrichtung, insbesondere des medizinischen Instruments, umfassen. Durch die beabstandete Anordnung des Verbindungselements zur Haltevorrichtung kann eine besonders geeignete Anordnung des Verbindungselements, insbesondere beabstandet zum medizinischen Instrument, ermöglicht werden.

Des Weiteren kann das Verbindungselement als Buchse und/oder Schaft und/oder, insbesondere elektronische, Schnittstelle ausgebildet sein. Ferner kann das Verbindungselement und/oder das Übertragungselement zumindest einen Bewegungssensor aufweisen, der dazu ausgebildet ist, eine Bewegung des Verbindungselements und/oder eine Bewegung des zumindest einen Teils der Haltevorrichtung zu erfassen. Dabei kann der Bewegungssensor beispielsweise einen optischen und/oder elektromagnetischen und/oder ultraschallbasierten Bewegungssensor umfassen. Hierdurch kann eine elektronische, insbesondere bidirektionale, Übertragung der Bewegung zwischen dem zumindest einen Teil der Haltevorrichtung, beispielsweise dem ersten und/oder dem zweiten Aufnahmeelement und/oder den zumindest drei Blendenelementen, und dem Verbindungselement ermöglicht.

Ferner kann die vorgeschlagene Haltevorrichtung einen weiteren Bewegungssensor aufweisen, der dazu ausgebildet ist, eine Bewegung des medizinischen Instruments relativ zur Haltevorrichtung, insbesondere relativ zu den zumindest drei Blendenelementen, zu erfassen. Dabei kann der weitere Bewegungssensor beispielsweise einen optischen und/oder elektromagnetischen und/oder ultraschallbasierten Bewegungssensor umfassen. Sofern das medizinische Instrument eine Markerstruktur und/oder zumindest ein Markerobjekt aufweist, kann der weitere Bewegungssensor vorteilhafterweise die Bewegung des medizinischen Instruments basierend auf der Bewegung der Markerstruktur und/oder des zumindest einen Markerobjekts erfassen.

In einer weiteren vorteilhaften Ausführungsform der vorgeschlagenen Bewegungsvorrichtung kann das Übertragungselement einen Riemenantrieb und/oder einen Zahnradantrieb aufweisen. Dabei kann der Riemenantrieb zumindest einen Riemen und/oder ein Riemenrad umfassen, wobei der Riemen eine Bewegung zwischen dem zumindest einen Teil der Haltevorrichtung und dem Riemenrad überträgt. Ferner kann der Zahnradantrieb zumindest ein Zahnrad aufweisen, wobei das Zahnrad mechanisch mit dem zumindest einem Teil der Haltevorrichtung gekoppelt sein kann. Vorteilhafterweise kann das Verbindungselement mechanisch und/oder elektronisch mit dem Riemenrad und/oder dem Zahnrad gekoppelt sein. Hierdurch kann eine besonders präzise und direkte Übertragung der Bewegung zwischen dem zumindest einem Teil der Haltevorrichtung und dem Verbindungselement ermöglicht werden.

In einer weiteren vorteilhaften Ausführungsform der vorgeschlagenen Bewegungsvorrichtung kann die Haltevorrichtung mehrere Bewegungsfreiheitsgrade aufweisen. Dabei kann das Übertragungselement die Bewegung gemäß der mehreren Bewegungsfreiheitsgrade zwischen der Haltevorrichtung und dem Verbindungselement, insbesondere bidirektional und/oder gleichzeitig, übertragen. Insbesondere kann das erste und/oder das zweite Aufnahmeelement und/oder die zumindest drei Blendenelemente jeweils zumindest einen Bewegungsfreiheitsgrad aufweisen. Beispielsweise kann das erste und/oder das zweite Aufnahmeelement einen Bewegungsfreiheitsgrad zur Rotation um die gemeinsame Rotationsachse aufweisen. Ferner können die zumindest drei Blendenelemente jeweils einen Bewegungsfreiheitsgrad innerhalb der zwangsgeführten Bewegung zwischen dem Öffnen und Schließen der Haltevorrichtung aufweisen.

Vorteilhafterweise umfasst das Übertragungselement mehrere Übertragungskanäle, beispielsweise Riemen und/oder Zahnräder und/oder Leitungen, die dazu ausgebildet sind, jeweils einen Bewegungsfreiheitsgrad der Haltevorrichtung zwischen der Haltevorrichtung und dem Verbindungselement zu übertragen. Vorteilhafterweise können die mehreren Bewegungsfreiheitsgrade der Haltevorrichtung parallel und/oder gleichzeitig und/oder unabhängig voneinander und/oder bidirektional durch das Übertragungselement übertragen werden. Hierdurch kann vorteilhafterweise eine einzelne und/oder gleichzeitige Ansteuerung der mehreren Bewegungsfreiheitsgrade der Haltevorrichtung durch das Verbindungselement ermöglicht werden. Ferner kann eine Erfassung einer Bewegung zumindest eines Teils der Haltevorrichtung korrespondierend zu jeweils einem Bewegungsfreiheitsgrad, insbesondere separat, durch das Verbindungselement erfasst werden. Dabei kann das Verbindungselement und/oder das Übertragungselement vorteilhafterweise jeweils einen Bewegungssensor zur Erfassung jeweils eines Bewegungsfreiheitsgrades der Haltevorrichtung umfassen.

In einer weiteren vorteilhaften Ausführungsform der vorgeschlagenen Bewegungsvorrichtung kann das Verbindungselement jeweils eine Verbindungsaufnahme zu jedem der Bewegungsfreiheitsgrade der Haltevorrichtung aufweisen. Dabei können die Verbindungsaufnahmen vorteilhafterweise als Buchse und/oder Schaft und/oder, insbesondere elektronische, Schnittstelle ausgebildet sein. Vorzugsweise kann zumindest eine der Verbindungsaufnahmen zur mechanischen und/oder elektronischen Kopplung eines Roboters und/oder eines medizinischen Geräts und/oder einer Verarbeitungseinheit ausgebildet sein.

In einer weiteren vorteilhaften Ausführungsform der vorgeschlagenen Bewegungsvorrichtung kann die Bewegungsvorrichtung weiterhin ein Gehäuse umfassen. Dabei kann das Gehäuse die Haltevorrichtung und das Übertragungselement derart zumindest teilweise umschließen, dass das medizinische Instrument in die Haltevorrichtung einbringbar ist. Vorteilhafterweise können die Haltevorrichtung und das Übertragungselement durch das Gehäuse vor äußerer mechanischer und/oder chemischer Einwirkung geschützt werden. Ferner kann eine hygienische Reinigung der Bewegungsvorrichtung durch das Gehäuse vereinfacht werden. Des Weiteren kann eine mechanische Stabilität der Anordnung umfassend die Haltevorrichtung, das Übertragungselement und das Verbindungselement durch das Gehäuse verbessert werden. Vorteilhafterweise kann das Gehäuse als sterile Barriere zwischen der Haltevorrichtung mit dem Übertragungselement und dem Verbindungselement ausgebildet sein. Insbesondere kann die Haltevorrichtung mit dem Verbindungselement durch das Gehäuse, insbesondere die sterile Barriere, hindurch an das Verbindungselement mechanisch und/oder elektromagnetisch gekoppelt sein.

In einer weiteren vorteilhaften Ausführungsform der vorgeschlagenen Bewegungsvorrichtung kann die Bewegungsvorrichtung weiterhin ein Befestigungselement umfassen. Dabei kann das Befestigungselement zur Befestigung der Bewegungsvorrichtung an einem medizinischen Gerät und/oder einer Patientenlagerungsvorrichtung ausgebildet sein. Ferner kann das Befestigungselement ein Stativ und/oder eine Schraubzwinge und/oder eine Klemmhalterung und/oder einen Roboterarm umfassen. Vorzugsweise kann die Bewegungsvorrichtung mittels des Befestigungselements an einem medizinischen Gerät, beispielsweise einem Interventionsroboter und/oder einem Roboterarm und/oder einem medizinischen Bildgebungsgerät, und/oder einer Patientenlagerungsvorrichtung, insbesondere beweglich, befestigt werden. Hierdurch kann eine Bewegung des in der Bewegungsvorrichtung angeordneten medizinischen Instruments relativ zur Bewegungsvorrichtung, insbesondere zum Befestigungselement, ermöglicht werden. Ferner kann die Bewegungsvorrichtung mittels des Befestigungselements beweglich, beispielsweise an einer Führungsschiene, des medizinischen Geräts und/oder der Patientenlagerungsvorrichtung befestigt sein.

In einer weiteren vorteilhaften Ausführungsform der vorgeschlagenen Bewegungsvorrichtung kann das Befestigungselement einen Motorantrieb aufweisen, der dazu ausgebildet ist, die Bewegungsvorrichtung zu bewegen. Dabei kann der Motorantrieb beispielsweise einen Elektromotor umfassen. Ferner kann der Motorantrieb beispielsweise als Rollenantrieb zur Bewegung der Bewegungsvorrichtung entlang einer Führungsschiene ausgebildet sein. Hierdurch kann eine besonders präzise und geführte Bewegung der Bewegungsvorrichtung, insbesondere des medizinischen Instruments, entlang einer Befestigung an dem medizinischen Gerät und/oder der Patientenlagerungsvorrichtung ermöglicht werden.

In einer weiteren vorteilhaften Ausführungsform der vorgeschlagenen Bewegungsvorrichtung kann die Bewegungsvorrichtung weiterhin ein Motorelement umfassen. Dabei kann das Motorelement dazu ausgebildet sein, mit dem Verbindungselement, insbesondere mechanisch, derart verbunden zu werden, dass das Motorelement und das Verbindungselement bewegungsgekoppelt sind. Hierbei kann das Motorelement vorteilhafterweise einen Motor, beispielsweise einen Elektromotor, umfassen. Ferner kann das Motorelement elektronisch und/oder mechanisch an das Verbindungselement gekoppelt sein. Hierdurch kann eine Bewegung zwischen dem Motorelement und zumindest einem Teil der Haltevorrichtung durch das Übertragungselement übertragbar sein. Ferner kann zwischen dem Motorelement und der der Haltevorrichtung mit dem Übertragungselement vorteilhafterweise eine sterile Barriere angeordnet sein. Dabei kann das Gehäuse die Haltevorrichtung und das Übertragungselement vorteilhafterweise zumindest teilweise umschließen und somit eine sterile Barriere gegenüber dem Motorelement und/oder dem Befestigungselement bilden.

Dabei kann das Motorelement sowohl zum Öffnen und/oder Schließen der Haltevorrichtung, als auch zur Bewegung der Haltevorrichtung als Ganzes ausgebildet sein. Hierdurch kann vorteilhafterweise eine Rotationsbewegung des in der Haltevorrichtung gehaltenen medizinischen Instruments ermöglicht werden.

In einer weiteren vorteilhaften Ausführungsform der vorgeschlagenen Bewegungsvorrichtung kann das Motorelement ein Sensorelement zur Erfassung einer Bewegung zumindest eines Teils der Haltevorrichtung umfassen. Dabei kann das Sensorelement beispielsweise einen optischen und/oder elektromagnetischen Sensor umfassen. Ferner kann eine Steuerung der Bewegung des zumindest einen Teils der Haltevorrichtung durch das Motorelement in Abhängigkeit der erfassten Bewegung des zumindest einen Teils der Haltevorrichtung und/oder eines anderen Teils der Haltevorrichtung erfolgen. Beispielsweise kann eine Rotationsbewegung der Haltevorrichtung als Ganzes in Abhängigkeit davon erfolgen, ob die Haltevorrichtung im geschlossenen und/oder geöffneten Zustand befindlich ist.

Durch die vorstehend beschriebenen Ausführungsformen der vorgeschlagenen Bewegungsvorrichtung kann vorteilhafterweise eine Unterstützung eines medizinischen Bedienpersonals, beispielsweise eines Arztes, bei einer Platzierung und/oder Führung und/oder Bewegung des medizinischen Instrument, insbesondere im Rahmen einer Intervention, ermöglicht werden.

Nachstehend wird ein Verfahren zur Bewegung eines medizinischen Instruments mittels einer ersten Bewegungsvorrichtung beschrieben. Die Vorteile des Verfahrens entsprechen im Wesentlichen den Vorteilen der vorab beschriebenen beispielhaften Bewegungsvorrichtung zur Bewegung eines medizinischen Instruments. Hierbei erwähnte Merkmale, Vorteile oder alternative Ausführungsformen können ebenso auch auf die anderen beanspruchten Gegenstände übertragen werden und umgekehrt.

Die Erfindung betrifft in einem weiteren Aspekt ein Verfahren zur Bewegung eines medizinischen Instruments mittels einer ersten Bewegungsvorrichtung. Dabei kann in einem Schritt r1) das medizinische Instrument in der ersten Bewegungsvorrichtung angeordnet werden. Insbesondere kann das medizinische Instrument in die Öffnung des ersten und des zweiten Aufnahmeelements der Haltevorrichtung der ersten Bewegungsvorrichtung eingebracht werden.

Ferner kann in einem Schritt r2) eine erste Position zumindest eines Abschnitts des medizinischen Instruments. Dabei kann beispielsweise eine Markierung und/oder ein Markerobjekt an dem medizinischen Instrument zur Bestimmung der ersten Position genutzt werden. Die erste Position kann vorzugsweise relativ zu einem Referenzpunkt im Raum und/oder zu einem Befestigungselement der Bewegungsvorrichtung und/oder relativ zu einem Untersuchungsobjekt und/oder relativ zu einem medizinischen Bildgebungsgerät bestimmt werden. Ferner kann die erste Position als Referenzposition des medizinischen Instruments nach dem Schließen der Haltevorrichtung bestimmt werden.

Des Weiteren kann in einem Schritt r3) das erste und/oder das zweite Aufnahmeelement der Haltevorrichtung der ersten Bewegungsvorrichtung derart bewegt werden, dass die zumindest drei Blendenelemente das medizinische Instrument halten. Dabei kann die Haltevorrichtung geschlossen werden. Vorteilhafterweise kann das medizinische Instrument nach Schritt r3) stabil und fest durch die zumindest drei Blendenelemente der Haltevorrichtung gehalten werden.

Ferner kann in einem Schritt r4) die erste Bewegungsvorrichtung aus einer Anfangsposition in eine Zielposition bewegt werden. Dabei kann die erste Bewegungsvorrichtung insbesondere als Ganzes rotiert und/oder verschoben werden. Ferner kann die Haltevorrichtung der ersten Bewegungsvorrichtung, insbesondere relativ zur ersten Bewegungsvorrichtung, rotiert werden. Dabei kann das medizinische Instrument zumindest abschnittsweise mitbewegt werden. Dies wird insbesondere durch das feste und stabile Halten des medizinischen Instruments in der Haltevorrichtung ermöglicht.

In einem Schritt r5) kann eine weitere Position des zumindest einen Abschnitts des medizinischen Instruments bestimmt werden. Dabei kann beispielsweise ein Rotationswinkel und/oder eine zurückgelegte Distanz und/oder eine Anzahl von Rotationen als weitere Position, insbesondere gegenüber der ersten Position und/oder der Referenzposition, bestimmt werden.

Hiernach kann in einem Schritt r6) das erste und/oder das zweite Aufnahmeelement der Haltevorrichtung der ersten Bewegungsvorrichtung derart bewegt werden, dass das medizinische Instrument von den zumindest drei Blendenelementen freigeben wird. Dabei wird die Haltevorrichtung vorteilhafterweise geöffnet und das medizinische Instrument wird freigegeben.

Dabei können die Schritte r2) bis r6) bis zum Erreichen einer Zielposition des zumindest einen Abschnitts des medizinischen Instruments, insbesondere iterativ, wiederholt werden. Hierdurch kann eine, insbesondere translatorische, Vorschubbewegung und/oder eine Rotationsbewegung des medizinischen Instruments, insbesondere über große Distanz, ermöglicht werden, wobei die Bewegungsvorrichtung wiederholt über eine vergleichsweise geringere Distanz bewegt wird.

Die Erfindung betrifft in einem weiteren Aspekt eine Koordinationsvorrichtung zur koordinierten Bewegung eines medizinischen Instruments. Dabei kann die Koordinationsvorrichtung vorteilhafterweise eine erste Bewegungsvorrichtung und zumindest eine weitere Bewegungsvorrichtung umfassen. Ferner können die erste und die zumindest eine weitere Bewegungsvorrichtung beabstandet voneinander angeordnet sein. Dabei kann die erste und die zumindest eine weitere Bewegungsvorrichtung zur Bewegung desselben medizinischen Instruments ausgebildet sein. Ferner können die erste und die zumindest eine weitere Bewegungsvorrichtung das medizinische Instrument koordiniert bewegen.

Dabei kann die erste und/oder die zumindest eine weitere Bewegungsvorrichtung insbesondere eine vorstehend beschriebene vorgeschlagene Bewegungsvorrichtung sein. Ferner können die erste und die zumindest eine weitere Bewegungsvorrichtung vorteilhafterweise derart voneinander beabstandet angeordnet sein, dass diese dasselbe medizinische Instrument aufnehmen können. Beispielsweise können die erste und die zumindest eine weitere Bewegungsvorrichtung entlang einer Längsrichtung des medizinischen Instruments angeordnet sein. Ferner können die erste und die zumindest eine weitere Bewegungsvorrichtung entlang eines gekrümmt verlaufenden medizinischen Instruments beanstandet voneinander angeordnet sein.

Des Weiteren können die erste und die zumindest eine weitere Bewegungsvorrichtung zur koordinierten Bewegung des medizinischen Instruments ausgebildet sein. Hierbei kann eine koordinierte Bewegung insbesondere eine auf einen Zustand des medizinischen Instruments und/oder einen Zustand der ersten und/oder der zumindest einen weiteren Bewegungsvorrichtung abgestimmte Bewegung und/oder einen abgestimmten Bewegungsablauf umfassen. Ferner kann die Koordination der Bewegung des medizinischen Instruments insbesondere in Abhängigkeit eines Zustands der ersten und/oder der zumindest einen weitere Bewegungsvorrichtung erfolgen. Ferner kann die Koordination der Bewegung des medizinischen Instruments in Abhängigkeit eines Zustands des medizinischen Instruments erfolgen. Vorteilhafterweise kann die Koordinationsvorrichtung eine Verarbeitungseinheit umfassen, die zur Koordination der Bewegung des medizinischen Instruments ausgebildet ist. Dabei kann die Verarbeitungseinheit vorzugsweise zur Steuerung der ersten und/oder der zumindest einen weiteren Bewegungsvorrichtung ausgebildet sein.

Ferner kann die Koordinationsvorrichtung einen Bewegungssensor aufweisen, der dazu ausgebildet ist, eine Bewegung des medizinischen Instruments relativ zur ersten und zur zumindest einen weiteren Bewegungsvorrichtung zu erfassen. Dabei kann der weitere Bewegungssensor beispielsweise einen optischen und/oder elektromagnetischen und/oder ultraschallbasierten Bewegungssensor umfassen. Beispielsweise kann der Bewegungssensor der Koordinationsvorrichtung als Mono-Kamera und/oder Stereo-Kamera ausgebildet sein. Sofern das medizinische Instrument eine Markerstruktur und/oder zumindest ein Markerobjekt aufweist, kann der weitere Bewegungssensor vorteilhafterweise die koordinierte Bewegung des medizinischen Instruments basierend auf der Bewegung der Markerstruktur und/oder des zumindest einen Markerobjekts erfassen. Dabei kann der Bewegungssensor beispielsweise an der ersten Bewegungsvorrichtung und/oder an der zumindest einen weiteren Bewegungsvorrichtung angeordnet sein.

In einer weiteren vorteilhaften Ausführungsform der vorgeschlagenen Koordinationsvorrichtung kann die Koordination der Bewegung des medizinischen Instruments durch die erste und/oder die zumindest eine weitere Bewegungsvorrichtung einen Ausgleich einer Torsion und/oder Verformung des medizinischen Instruments umfassen. Bei dem medizinischen Instrument kann es bei einer Bewegung, insbesondere Rotation und/oder Translation, zumindest eines Abschnitts des medizinischen Instruments zu einer Verformung, insbesondere durch Stauchung, und/oder Torsion des zumindest einen Abschnitts und/oder eines weiteren Abschnitts des medizinischen Instruments kommen. Vorteilhafterweise kann die Bewegung der ersten und/oder der zumindest einen weiteren Bewegungsvorrichtung derart koordiniert erfolgen, dass eine Torsion und/oder Verformung vermieden und/oder ausgeglichen wird.

Beispielsweise kann die erste Bewegungsvorrichtung in Abhängigkeit davon, ob die zumindest eine weitere Bewegungsvorrichtung geöffnet oder geschlossen ist, geöffnet oder geschlossen werden. Hierdurch kann eine freie Bewegung des medizinischen Instruments, das von der ersten und/oder der zumindest einen weiteren Bewegungsvorrichtung gehalten wird, durch die jeweils geöffnete Bewegungsvorrichtung hindurch ermöglicht werden. Ferner kann ein, insbesondere bekannter, Verlauf des chirurgischen Instruments zwischen der ersten und/oder der zumindest einen weiteren Bewegungsvorrichtung zur Koordination der Bewegung berücksichtigt werden. Beispielsweise kann ein zumindest abschnittsweiser gekrümmter und/oder geradliniger Verlauf des medizinischen Instruments bei einer Rotation der ersten und/oder der zumindest einen weiteren Bewegungsvorrichtung berücksichtigt werden. Ferner kann eine Relativposition der ersten und/oder der zumindest einen weiteren Bewegungsvorrichtung und/oder eine Distanz zwischen der ersten und der zumindest einen weiteren Bewegungsvorrichtung bei einer Bewegung des medizinischen Instruments berücksichtigt werden.

In einer weiteren vorteilhaften Ausführungsform der vorgeschlagenen Koordinationsvorrichtung kann die Koordinationsvorrichtung weiterhin eine Instrumentenerfassungseinheit umfassen. Dabei kann die Instrumentenerfassungseinheit zur Identifikation des in der ersten und der zumindest einen weiteren Bewegungsvorrichtung aufgenommenen medizinischen Instruments ausgebildet sein. Ferner kann die Bewegung des medizinischen Instruments durch die erste und/oder die zumindest eine weitere Bewegungsvorrichtung in Abhängigkeit einer Materialeigenschaft des identifizierten medizinischen Instruments erfolgen.

Dabei kann die Instrumentenerfassungseinheit beispielsweise eine Kamera, insbesondere eine Mono-Kamera und/oder eine Stereo-Kamera, und/oder einen Barcode-Scanner und/oder eine RFID-Erfassungseinheit und/oder eine elektromagnetische Antenneneinheit umfassen. Ferner kann das medizinische Instrument vorteilhafterweise zumindest eine identifizierende Eigenschaft, beispielsweise einen Barcode und/oder eine Markierung und/oder ein RFID-Kennzeichen und/oder eine elektromagnetische Sendeeinheit umfassen. Vorteilhafterweise kann das medizinische Instrument durch die Instrumentenerfassungseinheit identifiziert werden, sobald das medizinische Instrument in der ersten und/oder der zumindest einen weiteren Bewegungsvorrichtung angeordnet ist und/oder an diese angenähert wird.

Vorzugsweise kann anhand des identifizierten medizinischen Instruments eine Materialeigenschaft des medizinischen Instruments ableitbar sein. Dabei kann die Materialeigenschaft eine Dichteinformation und/oder eine Härteinformation und/oder eine Verformungseigenschaft und/oder eine Maßinformation des medizinischen Instruments umfassen. Vorzugsweise kann die Materialeigenschaft des identifizierten medizinischen Instruments bei der koordinierten Bewegung der ersten und/oder der zumindest einen weiteren Bewegungsvorrichtung berücksichtigt werden. Beispielsweise kann eine Bewegungsgeschwindigkeit und/oder eine Bewegungsamplitude der ersten und/oder der zumindest einen weiteren Bewegungsvorrichtung in Abhängigkeit der Materialeigenschaft des identifizierten medizinischen Instruments vorgebbar sein.

In einer weiteren vorteilhaften Ausführungsform der vorgeschlagenen Koordinationsvorrichtung kann die Koordinationsvorrichtung weiterhin eine Zustandserfassungseinheit umfassen. Dabei kann die Zustandserfassungseinheit zur Erfassung einer Verformung und/oder Torsion des medizinischen Instruments ausgebildet sein. Dabei kann die Bewegung des medizinischen Instruments durch die erste und/oder die zumindest eine weitere Bewegungsvorrichtung in Abhängigkeit der erfassten Verformung und/oder Torsion erfolgen.

Dabei kann das medizinische Instrument eine zur Bewegung der ersten Bewegungsvorrichtung entgegengerichtete Rückstellkraft auswirken. Beispielsweise kann das medizinische Instrument zumindest abschnittsweise an und/oder in einem Untersuchungsobjekt angeordnet sein. Dabei kann durch Reibungskräfte zwischen dem Untersuchungsobjekt und dem daran und/oder darin angeordneten Abschnitt des medizinischen Instruments eine Rückstellkraft auf das übrige medizinische Instrument ausgeübt werden. Hierdurch kann eine Torsion und/oder Verformung des medizinischen Instruments nach einer Bewegung der ersten Bewegungsvorrichtung, bei der das medizinische Instrument zumindest abschnittsweise mitbewegt wird, auftreten. Insbesondere bei einer weit beanstandeten Anordnung der ersten Bewegungsvorrichtung gegenüber der zumindest einen weiteren Bewegungsvorrichtung, kann es notwendig sein, eine auftretende Torsion und/oder Verformung des medizinischen Instruments durch eine koordinierte Bewegung der zumindest einen weiteren Bewegungsvorrichtung auszugleichen und/oder zu minimieren.

Hierbei kann die Zustandserfassungseinheit beispielsweise eine Kamera, insbesondere eine Mono-Kamera und/oder Stereo-Kamera, und/oder einen Ultraschallsensor und/oder einen elektromagnetischen Sensor und/oder einen optischen Sensor und/oder einen taktilen Sensor umfassen. Vorteilhafterweise ist die Zustandserfassung ausgebildet eine Verformung und/oder Torsion des medizinischen Instruments zu erfassen. Dabei kann die Verformung und/oder Torsion insbesondere dynamisch und/oder abschnittsweise, insbesondere zwischen der ersten und der zumindest einen weiteren Bewegungsvorrichtung, durch die Zustandserfassungseinheit erfassbar sein. Beispielsweise kann die Zustandserfassungseinheit an der ersten und/oder der zumindest einen weiteren Bewegungsvorrichtung angeordnet sein.

Dabei kann eine Torsion des medizinischen Instruments beispielsweise mittels eines taktilen Sensors erfassbar sein, insbesondere durch eine Rückstellkraft zwischen medizinischem Instrument und der jeweiligen Bewegungsvorrichtung. Ferner kann eine Verformung und/oder Torsion des medizinischen Instruments mittels eines optischen Sensors und/oder einer Kamera anhand einer Oberflächenveränderung des medizinischen Instruments und/oder einem zumindest abschnittsweise geänderten Verlauf des medizinischen Instruments erfassbar sein.

Des Weiteren kann die Bewegung des medizinischen Instruments durch die erste und/oder die zumindest eine weitere Bewegungsvorrichtung derart koordiniert erfolgen, dass die von der Zustandserfassungseinheit erfasste Verformung und/oder Torsion des medizinischen Instruments ausgeglichen und/oder minimiert wird. Ferner kann die durch die Zustandserfassungseinheit, insbesondere momentan, erfasste Verformung und/oder Torsion des medizinischen Instruments bei der Freigabe der ersten und/oder der zumindest einen weiteren Bewegungsvorrichtung, insbesondere der jeweiligen Haltevorrichtung, berücksichtigt werden. Beispielsweise kann eine Bewegung der ersten und/oder der zumindest einen weiteren Bewegungsvorrichtung, insbesondere ein Öffnen der jeweiligen Haltevorrichtung, in Abhängigkeit der erfassten Verformung und/oder Torsion des medizinischen Instruments sperrbar und/oder freigebbar sein.

Ferner kann die zumindest eine weitere Bewegungsvorrichtung vorteilhafterweise gleichgerichtet zur ersten Bewegungsvorrichtung bewegbar sein. Hierdurch kann eine, durch eine weit voneinander beabstandete Anordnung der ersten Bewegungsvorrichtung gegenüber der zumindest einen weiteren Bewegungsvorrichtung hervorgerufene, Torsion und/oder Verformung des medizinischen Instruments ausgeglichen und/oder minimiert werden. Vorteilhafterweise ist die zumindest eine weitere Bewegungsvorrichtung näher an dem Untersuchungsobjekt angeordnet, an und/oder in dem das medizinische Instrument zumindest abschnittsweise angeordnet ist, als die erste Bewegungsvorrichtung.

Hierdurch kann eine besonders materialschonende und sichere Bewegung des medizinischen Instruments durch die Koordinationsvorrichtung ermöglicht werden. Ferner kann ein Verletzungsrisiko für das Untersuchungsobjekt minimiert werden.

In einer weiteren vorteilhaften Ausführungsform der vorgeschlagenen Koordinationsvorrichtung kann die erste und die zumindest eine weitere Bewegungsvorrichtung mittels des jeweiligen Befestigungselements entlang zumindest einer gemeinsamen Bewegungsachse insbesondere konstant zueinander beanstandet, beweglich angeordnet sein. Dabei kann die zumindest eine gemeinsame Bewegungsachse vorteilhafterweise parallel zu einer Längsachse des medizinischen Instruments verlaufen. Ferner kann das Befestigungselement, insbesondere motorisiert, beweglich an ein Schienensystem mechanisch und/oder magnetisch koppelbar sein. Hierdurch kann eine gerichtete Bewegung der ersten und der zumindest einen weiteren Bewegungsvorrichtung, insbesondere der Koordinationsvorrichtung, ermöglicht werden.

Ferner kann eine, insbesondere translatorische, Vorschubbewegung und/oder eine Rotationsbewegung des medizinischen Instruments, insbesondere über große räumliche Distanz, ermöglicht werden, wobei die erste Bewegungsvorrichtung wiederholt, insbesondere iterativ, über eine vergleichsweise geringere Distanz relativ zur zumindest einen weiteren Bewegungsvorrichtung koordiniert bewegbar ist. Dabei kann die Verarbeitungseinheit vorteilhafterweise dazu ausgebildet sein, eine räumliche Positionierung der ersten Bewegungsvorrichtung und der zumindest einen weiteren Bewegungsvorrichtung, insbesondere relativ zueinander und/oder relativ zum Untersuchungsobjekt, zu erfassen. Dabei kann die räumliche Positionierung eine Information zur räumlichen Ausrichtung und/oder Position der ersten Bewegungsvorrichtung und der zumindest einen weiteren Bewegungsvorrichtung umfassen.

In einer weiteren vorteilhaften Ausführungsform der vorgeschlagenen Koordinationsvorrichtung kann die Koordinationsvorrichtung weiterhin ein Griffelement umfassen. Dabei kann die Anordnung der ersten und der zumindest einen weiteren Bewegungsvorrichtung entlang der zumindest einen gemeinsamen Bewegungsachse beweglich an dem Griffelement befestigt sein. Ferner kann das Griffelement von einem Bedienpersonal haltbar sein. Dabei kann das Griffelement vorteilhafterweise eine Halterung und/oder ein Gehäuse und/oder ein Schienensystem zur beweglichen Befestigung der ersten und der zumindest einen weiteren Bewegungsvorrichtung umfassen. Ferner kann das Griffelement als, insbesondere ergonomische, Ausbuchtung und/oder Aussparung und/oder Einbuchtung an einem Gehäuse der Koordinationsvorrichtung ausgebildet sein. Insbesondere kann das Griffelement die erste und die zumindest eine weitere Bewegungsvorrichtung zumindest teilweise umschließen. Hierdurch kann eine koordinierte Bewegung des medizinischen Instruments ermöglicht werden, während die Koordinationsvorrichtung mittels des Griffelements von einem Bedienpersonal gehalten wird. Dabei kann die zur Bewegung des medizinischen Instruments notwendige Rückstellkraft vorteilhafterweise durch das Bedienpersonal über das Griffelement auf die erste und/oder die zumindest eine weitere Bewegungsvorrichtung übertragbar sein.

Hierdurch kann ein besonders flexibler Einsatz der Koordinationsvorrichtung ermöglicht werden.

Durch die vorstehend beschriebenen Ausführungsformen der vorgeschlagenen Koordinationsvorrichtung kann vorteilhafterweise eine Unterstützung eines medizinischen Bedienpersonals bei einer Platzierung und/oder Führung und/oder Bewegung des medizinischen Instruments, insbesondere im Rahmen einer Intervention, ermöglicht werden.

Nachstehend wird ein Verfahren zur Koordination einer Bewegung eines medizinischen Instruments mittels einer Koordinationsvorrichtung. Die Vorteile des Verfahrens entsprechen im Wesentlichen den Vorteilen der vorab beschriebenen beispielhaften Koordinationsvorrichtung zur koordinierten Bewegung eines medizinischen Instruments. Hierbei erwähnte Merkmale, Vorteile oder alternative Ausführungsformen können ebenso auch auf die anderen beanspruchten Gegenstände übertragen werden und umgekehrt.

Die Erfindung betrifft in einem weiteren Aspekt ein Verfahren zur Koordination einer Bewegung eines medizinischen Instruments mittels einer Koordinationsvorrichtung. In einem Schritt s1) kann das medizinische Instrument in der ersten und der zumindest einen weiteren Bewegungsvorrichtung angeordnet werden. Dabei kann das medizinische Instrument insbesondere in die jeweilige Öffnung der Haltevorrichtung der ersten und der zumindest einen weiteren Bewegungsvorrichtung eingebracht werden. Ferner kann die erste und/oder die zumindest eine weitere Bewegungsvorrichtung derart an dem medizinischen Instrument angeordnet werden, dass das medizinische Instrument in der jeweiligen Öffnung der Haltevorrichtung aufgenommen ist.

Ferner kann in einem Schritt s2) eine erste Position zumindest eines Abschnitts des medizinischen Instruments bestimmt werden. Dabei kann beispielsweise eine Markierung und/oder ein Markerobjekt an dem medizinischen Instrument zur Bestimmung der ersten Position genutzt werden. Die erste Position kann vorzugsweise relativ zu einem Referenzpunkt im Raum und/oder relativ zur ersten Bewegungsvorrichtung und/oder relativ zur zumindest einen weiteren Bewegungsvorrichtung und/oder relativ zu einem Untersuchungsobjekt und/oder relativ zu einem medizinischen Bildgebungsgerät bestimmt werden. Ferner kann die erste Position als Referenzposition des medizinischen Instruments nach dem Schließen der Haltevorrichtung der ersten und/oder der zumindest einen weiteren Bewegungsvorrichtung bestimmt werden.

In einem Schritt s3) kann das erste und/oder das zweite Aufnahmeelement der Haltevorrichtung der ersten Bewegungsvorrichtung derart bewegt werden, dass die jeweils zumindest drei Blendenelemente das medizinische Instrument halten. Dabei kann die Haltevorrichtung der ersten Bewegungsvorrichtung geschlossen werden. Vorteilhafterweise kann das medizinische Instrument nach Schritt s3) stabil und fest durch die zumindest drei Blendenelemente der Haltevorrichtung der ersten Bewegungsvorrichtung gehalten werden.

Ferner kann in einem Schritt s4) eine Bewegung der ersten Bewegungsvorrichtung aus einer Anfangsposition in eine Zielposition erfolgen, wobei das medizinische Instrument zumindest abschnittsweise mitbewegt wird. Dabei kann die erste Bewegungsvorrichtung insbesondere als Ganzes rotiert und/oder verschoben werden. Dabei kann die Verschiebung der ersten Bewegungsvorrichtung insbesondere relativ zur zumindest einen weiteren Bewegungsvorrichtung erfolgen. Ferner kann die Haltevorrichtung der ersten Bewegungsvorrichtung, insbesondere relativ zur ersten Bewegungsvorrichtung, rotiert werden. Dabei kann das medizinische Instrument zumindest abschnittsweise mitbewegt werden. Dies wird insbesondere durch das feste und stabile Halten des medizinischen Instruments in der Haltevorrichtung der ersten Bewegungsvorrichtung ermöglicht. Vorteilhafterweise ist die zumindest eine weitere Bewegungsvorrichtung während der Bewegung der ersten Bewegungsvorrichtung geöffnet. Hierdurch kann erreicht werden, dass das medizinische Instrument während der Bewegung der ersten Bewegungsvorrichtung in der zumindest einen weiteren Bewegungsvorrichtung frei beweglich aufgenommen ist.

In einem Schritt s5) kann eine weitere Position des zumindest einen Abschnitts des medizinischen Instruments relativ zur ersten Bewegungsvorrichtung bestimmt werden. Dabei kann beispielsweise ein Rotationswinkel und/oder eine zurückgelegte Distanz und/oder eine Anzahl von Rotationen als weitere Position, insbesondere gegenüber der ersten Position und/oder der Referenzposition, bestimmt werden.

Des Weiteren kann in einem Schritt s6) das erste und/oder das zweite Aufnahmeelement der Haltevorrichtung der zumindest einen weiteren Bewegungsvorrichtung derart bewegt werden, dass die jeweils zumindest drei Blendenelemente das medizinische Instrument halten. Dabei kann die Haltevorrichtung der zumindest einen weiteren Bewegungsvorrichtung geschlossen werden. Vorteilhafterweise kann das medizinische Instrument nach Schritt s6) stabil und fest durch die zumindest drei Blendenelemente der Haltevorrichtung der zumindest einen weiteren Bewegungsvorrichtung gehalten werden.

Ferner kann in einem Schritt s7) das erste und/oder das zweite Aufnahmeelement der Haltevorrichtung der ersten Bewegungsvorrichtung derart bewegt werden, dass das medizinische Instrument durch die zumindest drei Blendenelemente freigegeben wird. Dabei kann die Haltevorrichtung der ersten Bewegungsvorrichtung geöffnet werden. Vorteilhafterweise kann das medizinische Instrument nach Schritt s7) frei beweglich in der ersten Bewegungsvorrichtung aufgenommen sein.

Hiernach kann in einem Schritt s8) eine Bewegung der ersten Bewegungsvorrichtung aus der Zielposition in eine weitere Anfangsposition erfolgen. Vorteilhafterweise kann die weitere Anfangsposition der Anfangsposition der ersten Bewegungsvorrichtung aus Schritt s4) entsprechen. Ferner kann die weitere Anfangsposition als Anfangsposition in Schritt s4) vorgegeben werden. Des Weiteren können die Schritte s1) bis s8) bis zum Erreichen einer Zielposition des zumindest einen Abschnitts des medizinischen Instruments, insbesondere iterativ, wiederholt werden. Hierdurch kann eine Vorschubbewegung und/oder eine Rotationsbewegung des medizinischen Instruments, insbesondere über große Distanz, ermöglicht werden, wobei die erste Bewegungsvorrichtung wiederholt, insbesondere iterativ, über eine vergleichsweise geringere Distanz bewegt wird. Ferner kann die Bewegung der ersten und der zumindest einen weiteren Bewegungsvorrichtung derart koordiniert werden, dass ein Öffnen und Schließen der jeweiligen Haltevorrichtung in Abhängigkeit der jeweils anderen Haltevorrichtung erfolgt. Hierdurch kann vorteilhafterweise ein sicheres Halten des medizinischen Instruments, insbesondere zwischen zwei Bewegungsschritten, innerhalb der Koordinationsvorrichtung sichergestellt werden.

Nachstehend wird ein Verfahren zur optimierten Bewegung eines medizinischen Instruments mittels einer Koordinationsvorrichtung. Die Vorteile des Verfahrens entsprechen im Wesentlichen den Vorteilen der vorab beschriebenen beispielhaften Koordinationsvorrichtung zur koordinierten Bewegung eines medizinischen Instruments. Hierbei erwähnte Merkmale, Vorteile oder alternative Ausführungsformen können ebenso auch auf die anderen beanspruchten Gegenstände übertragen werden und umgekehrt.

Die Erfindung betrifft in einem weiteren Aspekt ein Verfahren zur optimierten Bewegung eines medizinischen Instruments mittels einer Koordinationsvorrichtung. Dabei kann die Koordinationsvorrichtung eine Zustandserfassungseinheit umfassen, die dazu ausgebildet ist, einen Zustand einer Torsion und/oder Verformung des medizinischen Instruments zu erfassen. Hierbei kann die Zustandserfassungseinheit beispielsweise eine Kamera, insbesondere eine Mono-Kamera und/oder Stereo-Kamera, und/oder einen Ultraschallsensor und/oder einen elektromagnetischen Sensor und/oder einen optischen Sensor und/oder einen taktilen Sensor umfassen. Vorteilhafterweise ist die Zustandserfassung ausgebildet eine Verformung und/oder Torsion des medizinischen Instruments zu erfassen. Dabei kann die Verformung und/oder Torsion insbesondere dynamisch und/oder abschnittsweise, insbesondere zwischen der ersten und der zumindest einen weiteren Bewegungsvorrichtung, durch die Zustandserfassungseinheit erfassbar sein. Beispielsweise kann die Zustandserfassungseinheit an der ersten und/oder der zumindest einen weiteren Bewegungsvorrichtung angeordnet sein.

In einem ersten Schritt t1) kann das medizinische Instrument in der ersten und der zumindest einen weiteren Bewegungsvorrichtung angeordnet werden. Dabei kann das medizinische Instrument insbesondere in die jeweilige Öffnung der Haltevorrichtung der ersten und der zumindest einen weiteren Bewegungsvorrichtung eingebracht werden. Ferner kann die erste und/oder die zumindest eine weitere Bewegungsvorrichtung derart an dem medizinischen Instrument angeordnet werden, dass das medizinische Instrument in der jeweiligen Öffnung der Haltevorrichtung aufgenommen ist.

Ferner kann in einem Schritt t2) eine Bewegung des ersten und/oder des zweiten Aufnahmeelements der Haltevorrichtung der ersten und der zumindest einen weiteren Bewegungsvorrichtung derart erfolgen, dass die jeweils zumindest drei Blendenelemente das medizinische Instrument halten. Dabei kann die jeweilige Haltevorrichtung der ersten und der zumindest einen weiteren Bewegungsvorrichtung geschlossen werden. Vorteilhafterweise kann das medizinische Instrument nach Schritt s3) stabil und fest durch die jeweils zumindest drei Blendenelemente der Haltevorrichtung der ersten und der zumindest einen weiteren Bewegungsvorrichtung gehalten werden.

In einem Schritt t3) kann ein Anfangszustand der Torsion und/oder Verformung des medizinischen Instruments mittels der Zustandserfassungseinheit erfasst werden. Dabei kann der Anfangszustand der Torsion und/oder Verformung des medizinischen Instruments vorteilhafterweise eine, insbesondere momentane, Materialeigenschaft des medizinischen Instruments umfassen. Ferner kann der Anfangszustand der Torsion beispielsweise durch einen Abgleich zwischen zumindest zwei Sensoren der Zustandserfassungseinheit bestimmt werden. Dabei kann vorzugsweise jeweils ein Sensor der Zustandserfassungseinheit an der ersten und der zumindest einen weiteren Bewegungsvorrichtung angeordnet sein.

In einem Schritt t4) kann die erste Bewegungsvorrichtung aus einer Anfangsposition in eine Zielposition bewegt werden. Dabei kann das medizinische Instrument zumindest abschnittsweise mitbewegt werden. Dabei kann die erste Bewegungsvorrichtung insbesondere als Ganzes rotiert und/oder verschoben werden. Dabei kann die Verschiebung der ersten Bewegungsvorrichtung insbesondere relativ zur zumindest einen weiteren Bewegungsvorrichtung erfolgen. Ferner kann die Haltevorrichtung der ersten Bewegungsvorrichtung, insbesondere relativ zur ersten Bewegungsvorrichtung, rotiert werden. Dabei kann das medizinische Instrument zumindest abschnittsweise mitbewegt werden. Dies wird insbesondere durch das feste und stabile Halten des medizinischen Instruments in der Haltevorrichtung der ersten Bewegungsvorrichtung ermöglicht.

Ferner kann in einem Schritt t5) ein Zwischenzustand der Torsion und/oder Verformung des medizinischen Instruments mittels der Zustandserfassungseinheit erfasst werden. Dabei kann der Zwischenzustand der Torsion und/oder Verformung des medizinischen Instruments vorteilhafterweise eine, insbesondere momentane, Materialeigenschaft des medizinischen Instruments umfassen. Ferner kann der Zwischenzustand der Torsion beispielsweise durch einen Abgleich zwischen zumindest zwei Sensoren der Zustandserfassungseinheit bestimmt werden. Dabei kann vorzugsweise jeweils ein Sensor der Zustandserfassungseinheit an der ersten und der zumindest einen weiteren Bewegungsvorrichtung angeordnet sein. Des Weiteren kann der Zwischenzustand der Torsion und/oder Verformung des medizinischen Instruments eine Information zu einer Veränderung und/oder Abweichung der Torsion und/oder Verformung des medizinischen Instruments gegenüber dem Anfangszustand umfassen.

In einem Schritt t6) kann die zumindest eine weitere Bewegungsvorrichtung in Abhängigkeit zur Bewegung der ersten Bewegungsvorrichtung in Schritt t4) erfolgen. Dabei kann das medizinische Instrument zumindest abschnittsweise mitbewegt werden. Dabei kann die zumindest eine weitere Bewegungsvorrichtung insbesondere als Ganzes rotiert und/oder verschoben werden. Ferner kann die Verschiebung der zumindest einen weiteren Bewegungsvorrichtung insbesondere relativ zur ersten Bewegungsvorrichtung erfolgen. Ferner kann die Haltevorrichtung der zumindest einen weiteren Bewegungsvorrichtung, insbesondere relativ zur zumindest einen weiteren Bewegungsvorrichtung, rotiert werden. Dabei kann das medizinische Instrument zumindest abschnittsweise mitbewegt werden. Dies wird insbesondere durch das feste und stabile Halten des medizinischen Instruments in der Haltevorrichtung der zumindest einen weiteren Bewegungsvorrichtung ermöglicht.

Ferner kann in einem Schritt t7) ein weiterer Zwischenzustand der Torsion und/oder Verformung des medizinischen Instruments mittels der Zustandserfassungseinheit erfasst werden. Zudem kann durch die Bewegung der zumindest einen weiteren Bewegungsvorrichtung in Schritt t6) eine Abweichung zwischen dem weiteren Zwischenzustand und dem Anfangszustand minimiert werden.

Dabei kann die zumindest eine weitere Bewegungsvorrichtung vorteilhafterweise gleichgerichtet zur ersten Bewegungsvorrichtung bewegt werden. Hierdurch kann eine, durch eine weit voneinander beabstandete Anordnung der ersten Bewegungsvorrichtung gegenüber der zumindest einen weiteren Bewegungsvorrichtung hervorgerufene, Torsion und/oder Verformung des medizinischen Instruments ausgeglichen und/oder minimiert werden. Vorteilhafterweise ist die zumindest eine weitere Bewegungsvorrichtung näher an einem Untersuchungsobjekt angeordnet, welches durch das medizinische Instrument beeinflussbar ist, als die erste Bewegungsvorrichtung.

Die vorgeschlagenen Verfahren können insbesondere zur Unterstützung eines Bedienpersonals bei einer Bewegung und/oder Führung und/oder Ausrichtung eines medizinischen Instruments dienlich sein.

Ferner wird ein Computerprogrammprodukt vorgeschlagen, das ein Programm umfasst und direkt in einen Speicher einer programmierbaren Recheneinheit ladbar ist und Programmmittel, z.B. Bibliotheken und Hilfsfunktionen, aufweist, um ein vorgeschlagenes Verfahren auszuführen, wenn das Computerprogrammprodukt ausgeführt wird. Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Verarbeitungseinheit zu laden ist. Durch das Computerprogrammprodukt kann das ein vorgeschlagenes Verfahren schnell, identisch wiederholbar und robust ausgeführt werden. Das Computerprogrammprodukt ist so konfiguriert, dass es mittels der Verarbeitungseinheit die erfindungsgemäßen Verfahrensschritte ausführen kann. Die Verarbeitungseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer Verarbeitungseinheit geladen werden kann, der mit der Verarbeitungseinheit direkt verbunden oder als Teil der Verarbeitungseinheit ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Verarbeitungseinheit ein erfindungsgemäßes Verfahren durchführen. Beispiele für elektronisch lesbare Datenträger sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Verarbeitungseinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere vorteilhafte Ausführungsformen der Erfindung sind im Folgenden beschrieben:
Ausführungsform A1: Bewegungsvorrichtung zur Bewegung eines medizinischen Instruments umfassend eine Haltevorrichtung, ein Übertragungselement und ein Verbindungselement,
   wobei die Haltevorrichtung zum Halten des medizinischen Instruments ausgebildet ist,
   wobei das Übertragungselement eine Bewegung zwischen zumindest einem Teil der Haltevorrichtung und dem Verbindungselement, insbesondere bidirektional, überträgt,
   wobei das Verbindungselement zur Haltevorrichtung beanstandet angeordnet ist.
Ausführungsform A2: Bewegungsvorrichtung gemäß Ausführungsform A1, dadurch gekennzeichnet, dass das Übertragungselement zumindest einen Riemenantrieb und/oder Zahnradantrieb aufweist.
Ausführungsform A3: Bewegungsvorrichtung nach Ausführungsform A1 oder A2, dadurch gekennzeichnet, dass die Haltevorrichtung mehrere Bewegungsfreiheitsgrade aufweist,
   wobei das Übertragungselement die Bewegung gemäß der mehreren Bewegungsfreiheitsgrade zwischen der Haltevorrichtung und dem Verbindungselement, insbesondere bidirektional und/oder gleichzeitig, überträgt.
Ausführungsform A4: Bewegungsvorrichtung nach Ausführungsform A3, dadurch gekennzeichnet, dass das Verbindungselement jeweils eine Verbindungsaufnahme zu jedem der Bewegungsfreiheitsgrade der Haltevorrichtung aufweist.
Ausführungsform A5: Bewegungsvorrichtung nach einer der Ausführungsformen A1 bis A4 weiterhin umfassend ein Gehäuse, wobei das Gehäuse die Haltevorrichtung und das Übertragungselement derart umschließt, dass das medizinische Instrument in die Haltevorrichtung einbringbar ist.
Ausführungsform A6: Bewegungsvorrichtung nach einer der Ausführungsformen A1 bis A5, weiterhin umfassend ein Befestigungselement, wobei das Befestigungselement zur Befestigung der Bewegungsvorrichtung an einem medizinischen Gerät und/oder einer Patientenlagerungsvorrichtung ausgebildet ist.
Ausführungsform A7: Bewegungsvorrichtung nach Ausführungsform A6, wobei das Befestigungselement einen Motorantrieb aufweist, der dazu ausgebildet ist, die Bewegungsvorrichtung zu bewegen.
Ausführungsform A8: Bewegungsvorrichtung nach Ausführungsform A6 oder A7, weiterhin umfassend ein Motorelement, wobei das Motorelement dazu ausgebildet ist, mit dem Verbindungselement, insbesondere mechanisch, derart verbunden zu werden, dass das Motorelement und das Verbindungselement bewegungsgekoppelt sind.
Ausführungsform A9: Bewegungsvorrichtung nach einer der Ausführungsformen A6 bis A8, dadurch gekennzeichnet, dass das Motorelement ein Sensorelement zur Erfassung einer Bewegung zumindest eines Teils der Haltevorrichtung umfasst.
Ausführungsform V1: Verfahren zur Bewegung eines medizinischen Instruments mittels einer ersten Bewegungsvorrichtung nach einer der Ausführungsformen A1 bis A9, umfassend:
   r1) Anordnen des medizinischen Instruments in der ersten Bewegungsvorrichtung,
   r2) Bestimmen einer ersten Position zumindest eines Abschnitts des medizinischen Instruments,
   r3) Bewegen des ersten und/oder des zweiten Aufnahmeelements der Haltevorrichtung der ersten Bewegungsvorrichtung derart, dass die zumindest drei Blendenelemente das medizinische Instrument halten,
   r4) Bewegen der ersten Bewegungsvorrichtung aus einer Anfangsposition in eine Zielposition, wobei das medizinische Instrument zumindest abschnittsweise mitbewegt wird,
   r5) Bestimmen einer weiteren Position des zumindest einen Abschnitts des medizinischen Instruments,
   r6) Bewegen des ersten und/oder des zweiten Aufnahmeelements der Haltevorrichtung der ersten Bewegungsvorrichtung derart, dass das medizinische Instrument von den zumindest drei Blendenelementen freigegeben wird,
   wobei die Schritte r2) bis r6) bis zum Erreichen einer Zielposition des zumindest einen Abschnitts des medizinischen Instruments wiederholt werden.
Ausführungsform B1: Koordinationsvorrichtung zur koordinierten Bewegung eines medizinischen Instruments, umfassend eine erste Bewegungsvorrichtung, beispielsweise nach einer der Ausführungsformen A1 bis A8, und zumindest eine weitere Bewegungsvorrichtung, beispielsweise nach einer der Ausführungsformen A1 bis A8,
   wobei die erste und die zumindest eine weitere Bewegungsvorrichtung beabstandet voneinander angeordnet sind,
   wobei die erste und die zumindest eine weitere Bewegungsvorrichtung zur Bewegung desselben medizinischen Instruments ausgebildet sind,
   wobei die erste und die zumindest eine weitere Bewegungsvorrichtung das medizinische Instrument koordiniert bewegen.
Ausführungsform B2 Koordinationsvorrichtung nach Ausführungsform B1, wobei die Koordination der Bewegung des medizinischen Instruments durch die erste und/oder die zumindest eine weitere Bewegungsvorrichtung einen Ausgleich einer Torsion und/oder Verformung des medizinischen Instruments umfasst.
Ausführungsform B3: Koordinationsvorrichtung nach Ausführungsform B2, weiterhin umfassend eine Instrumentenerfassungseinheit, die zur Identifikation des in der ersten und der zumindest einen weiteren Bewegungsvorrichtung aufgenommenen medizinischen Instruments ausgebildet ist,
   die Bewegung des medizinischen Instruments durch die erste und/oder die zumindest eine weitere Bewegungsvorrichtung in Abhängigkeit einer Materialeigenschaft des identifizierten medizinischen Instruments erfolgt.
Ausführungsform B4: Koordinationsvorrichtung nach Ausführungsform B2 oder B3, weiterhin umfassend eine Zustandserfassungseinheit, wobei die Zustandserfassungseinheit zur Erfassung einer Verformung und/oder Torsion des medizinischen Instruments ausgebildet ist,
   wobei die Bewegung des medizinischen Instruments durch die erste und/oder die zumindest eine weitere Bewegungsvorrichtung in Abhängigkeit der erfassten Verformung und/oder Torsion erfolgt.
Ausführungsform B5: Koordinationsvorrichtung nach einer der Ausführungsformen B1 bis B4, umfassend die erste und die zumindest eine weitere Bewegungsvorrichtung nach einer der Ausführungsformen A6 bis A8,
   wobei die erste und die zumindest eine weitere Bewegungsvorrichtung entlang zumindest einer gemeinsamen Bewegungsachse, insbesondere konstant zueinander beabstandet, beweglich angeordnet sind.
Ausführungsform B6: Koordinationsvorrichtung nach Ausführungsform B5, weiterhin umfassend ein Griffelement, wobei die Anordnung der ersten und der zumindest einen weiteren Bewegungsvorrichtung entlang der zumindest einen gemeinsamen Bewegungsachse beweglich an dem Griffelement befestigt sind, wobei das Griffelement von einem Bedienpersonal haltbar ist.
Ausführungsform W1: Verfahren zur Koordination einer Bewegung eines medizinischen Instruments mittels einer Koordinationsvorrichtung, beispielsweise nach einer der Ausführungsformen B1 bis B4, umfassend:
   s1) Anordnen des medizinischen Instruments in der ersten und der zumindest einen weiteren Bewegungsvorrichtung,
   s2) Bestimmen einer ersten Position zumindest eines Abschnitts des medizinischen Instruments,
   s3) Bewegen des ersten und/oder des zweiten Aufnahmeelements der Haltevorrichtung der ersten Bewegungsvorrichtung derart, dass die jeweils zumindest drei Blendenelemente das medizinische Instrument halten,
   s4) Bewegen der ersten Bewegungsvorrichtung aus einer Anfangsposition in eine Zielposition, wobei das medizinische Instrument zumindest abschnittsweise mitbewegt wird,
   s5) Bestimmen einer weiteren Position des zumindest einen Abschnitts des medizinischen Instruments,
   s6) Bewegen des ersten und/oder des zweiten Aufnahmeelements der Haltevorrichtung der zumindest einen weiteren Bewegungsvorrichtung derart,
   dass die jeweils zumindest drei Blendenelemente das medizinische Instrument halten,
   s7) Bewegen des ersten und/oder des zweiten Aufnahmeelements der Haltevorrichtung der ersten Bewegungsvorrichtung derart, dass das medizinische Instrument durch die zumindest drei Blendenelemente freigegeben wird,
   s8) Bewegen der ersten Bewegungsvorrichtung aus der Zielposition in eine weitere Anfangsposition, wobei die weitere Anfangsposition in Schritt s4) als Anfangsposition vorgegeben wird,
   wobei die Schritte s1) bis s8) bis zum Erreichen einer Zielposition des zumindest einen Abschnitts des medizinischen Instruments wiederholt werden.
Ausführungsform W2: Verfahren zur optimierten Bewegung eines medizinischen Instruments mittels einer Koordinationsvorrichtung, beispielsweise nach einer der Ausführungsformen B1 bis B4,
   wobei die Koordinationsvorrichtung eine Zustandserfassungseinheit umfasst, die dazu ausgebildet ist, einen Zustand einer Torsion und/oder Verformung des medizinischen Instruments zu erfassen,
   umfassend:
      t1) Anordnen des medizinischen Instruments in der ersten und der zumindest einen weiteren Bewegungsvorrichtung,
      t2) Bewegen des ersten und/oder des zweiten Aufnahmeelements der Haltevorrichtung der ersten und der zumindest einen weiteren Bewegungsvorrichtung derart,
      dass die jeweils zumindest drei Blendenelemente das medizinische Instrument halten,
      t3) Erfassen eines Anfangszustands der Torsion und/oder Verformung des medizinischen Instruments mittels der Zustandserfassungseinheit,
      t4) Bewegen der ersten Bewegungsvorrichtung aus einer Anfangsposition in eine Zielposition, wobei das medizinische Instrument zumindest abschnittsweise mitbewegt wird,
      t5) Erfassen eines Zwischenzustands der Torsion und/oder Verformung des medizinischen Instruments mittels der Zustandserfassungseinheit,
      t6) Bewegen der zumindest einen weiteren Bewegungsvorrichtung in Abhängigkeit zur Bewegung der ersten Bewegungsvorrichtung in Schritt t4) und/oder zum erfassten Zwischenzustand,
      wobei das medizinische Instrument zumindest abschnittsweise mitbewegt wird,
      t7) Erfassen eines weiteren Zwischenzustands der Torsion und/oder Verformung des medizinischen Instruments mittels der Zustandserfassungseinheit,
      wobei durch die Bewegung in Schritt t6) eine Abweichung zwischen dem weiteren Zwischenzustand und dem Anfangszustand minimiert wird.
Ausführungsform C: Computerprogrammprodukt, welches ein Programm umfasst und direkt in einen Speicher einer programmierbaren Recheneinheit einer Verarbeitungseinheit ladbar ist, mit Programmmitteln um ein Verfahren nach Ausführungsform V1 und/oder W1 und/oder W2 auszuführen, wenn das Programm in der Recheneinheit der Verarbeitungseinheit ausgeführt wird.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet. Es zeigen
- Fig. 1 bis 10: schematische Darstellungen verschiedener Ausführungsformen einer vorgeschlagenen Haltevorrichtung;
- Fig. 11 und 12: eine schematische Darstellung einer geöffneten und einer geschlossenen Haltevorrichtung;
- Fig. 13: eine schematische Darstellung einer vorgeschlagenen Haltevorrichtung mit einem motorisierten Antriebselement;
- Fig. 14: eine schematische Darstellung einer vorgeschlagenen Haltevorrichtung mit einem Blendenfeststellelement;
- Fig. 15: eine schematische Darstellung einer vorgeschlagenen Bewegungsvorrichtung;
- Fig. 16: eine schematische Darstellung einer vorgeschlagenen Bewegungsvorrichtung mit Gehäuse;
- Fig. 17: eine schematische Darstellung eines Motorelements und eines Befestigungselements;
- Fig. 18: eine schematische Darstellung einer vorgeschlagenen Bewegungsvorrichtung mit Gehäuse und Motorelement;
- Fig. 19: eine schematische Darstellung eines Ablaufdiagramms eines Verfahrens zur Bewegung eines medizinischen Instruments mittels einer Bewegungsvorrichtung;
- Fig. 20: eine schematische Darstellung einer vorgeschlagenen Koordinationsvorrichtung zur koordinierten Bewegung eines medizinischen Instruments;
- Fig. 21: eine schematische Darstellung einer weiteren Ausführungsform einer vorgeschlagenen Koordinationsvorrichtung;
- Fig. 22: eine schematische Darstellung einer vorgeschlagenen Koordinationsvorrichtung mit Griffelement;
- Fig. 23: eine schematische Darstellung eines Ablaufdiagramms eines Verfahrens zur Koordination einer Bewegung eines medizinischen Instruments mittels einer Koordinationsvorrichtung;
- Fig. 24: eine schematische Darstellung eines Ablaufdiagramms eines Verfahrens zur optimierten Bewegung eines medizinischen Instruments mittels einer Koordinationsvorrichtung.

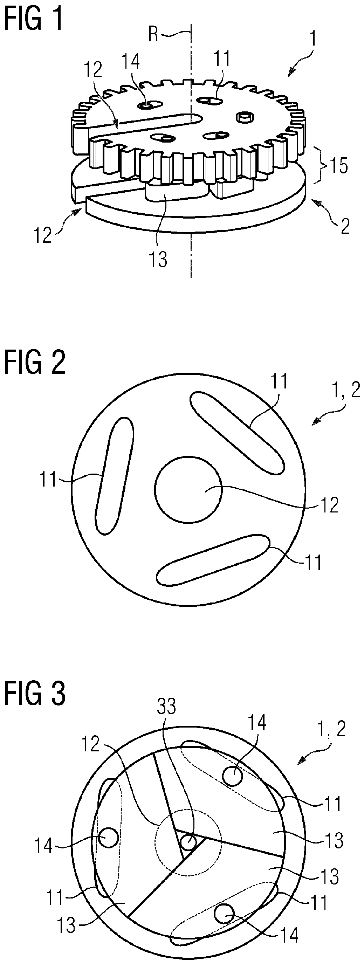

In Fig. 1 ist eine Ausführungsform der vorgeschlagenen Haltevorrichtung schematisch dargestellt. Dabei kann die Haltevorrichtung ein erstes Aufnahmeelement 1, ein zweites Aufnahmeelement 2 und zumindest drei Blendenelemente 13 umfassen. Ferner können die zumindest drei Blendenelemente 13 innerhalb einer Blendenschicht 15 zwischen dem ersten 1 und dem zweiten Aufnahmeelement 2 um eine gemeinsame Rotationsachse R herum angeordnet sind. Dabei kann die gemeinsame Rotationsachse R vorteilhafterweise im Wesentlichen senkrecht, insbesondere nicht parallel, zur Blendenschicht 15 verlaufen. Dabei kann das erste 1 und das zweite Aufnahmeelement 2 jeweils eine Öffnung 12 zur Aufnahme des medizinischen Instruments aufweisen (hier nicht gezeigt). Ferner können das erste 1 und das zweite Aufnahmeelement 2 um die gemeinsame Rotationsachse R herum relativ zueinander beweglich sein. Zudem können die zumindest drei Blendenelemente 13 jeweils zumindest ein erstes Kopplungselement 14 umfassen. Ferner kann das erste 1 und/oder das zweite Aufnahmeelement 2 zu jedem der zumindest drei Blendenelemente 13 jeweils zumindest ein zweites Kopplungselement 11 umfassen. Dabei kann jedes der zumindest drei Blendenelemente 13 innerhalb der Blendenschicht 15, in der diese angeordnet sind, durch mechanische Kopplung zwischen dem jeweiligen zumindest einen ersten Kopplungselement 14 des Blendenelements 13 und dem zumindest einen zweiten Kopplungselement 11 zwangsgeführt sein. Ferner kann bei einer Bewegung des ersten Aufnahmeelements 1 relativ zum zweiten Aufnahmeelement 2 um die gemeinsame Rotationsachse R herum eine durch das erste 1 und das zweite Aufnahmeelement 2 derart zwangsgeführte Bewegung der zumindest drei Blendenelemente 13 innerhalb der Blendenschicht 15 hin zur Öffnung 12 zur Aufnahme des medizinischen Instruments erfolgen, dass die zumindest drei Blendenelemente 13 ein in der Öffnung 12 des ersten 1 und des zweiten Aufnahmeelements 2 angeordnetes medizinisches Instrument halten.

Im in Fig. 1 schematisch dargestellten Ausführungsbeispiel umfasst die Haltevorrichtung beispielhaft fünf Blendenelemente 13. Dabei kann das das erste Aufnahmeelement 1 zu jedem der zumindest drei Blendenelemente 13 jeweils ein zweites Kopplungselement 11 umfassen, das als längliche Führung ausgespart ist und geradlinig verläuft. Ferner kann das zweite Aufnahmeelement 2 zu jedem der zumindest drei Blendenelemente 13 jeweils ein zweites Kopplungselement (hier nicht gezeigt) umfassen, das als längliche Führung ausgespart ist und gekrümmt verläuft. Ferner können das erste 1 und das zweite Aufnahmeelement 2 radförmig ausgebildet sein. Dabei kann das beispielhaft das erste Aufnahmeelement 1 als Zahnrad ausgebildet sein. Ferner kann die Öffnung 12 zur Aufnahme des medizinischen Instruments des ersten 1 und des zweiten Aufnahmeelements 2 als Schlitz ausgebildet sein und von der gemeinsamen Rotationsachse R begrenzt werden. Ferner können die zumindest drei Blendenelemente 13 eine dreieckige Form aufweisen. Dabei kann jedes der zumindest drei Blendenelemente 13 ferner jeweils zwei erste Kopplungselemente 14 aufweisen, die als stiftförmige Erhebungen ausgebildet sein können. Dabei kann jeweils ein erstes der zwei ersten Kopplungselemente 14 von jedem der zumindest drei Blendenelemente 13 auf einer dem ersten Aufnahmeelement 1 zugewandten Seite des Blendenelements 13 angeordnet sein. Ferner kann jeweils ein zweites der zwei ersten Kopplungselemente 14 von jedem der zumindest drei Blendenelemente 13 auf einer dem zweiten Aufnahmeelement 2 zugewandten Seite des Blendenelements 13 angeordnet sein. Dabei können die zwei ersten Kopplungselemente 14 von jedem der zumindest drei Blendenelemente 13 vorteilhafterweise nicht auf einer zur gemeinsamen Rotationsachse R parallelen Raumachse liegen.

Dabei kann die vorgeschlagene Haltevorrichtung vorteilhafterweise platzsparend und/oder mit modularen Komponenten ausgebildet sein. Ferner kann die Haltevorrichtung zum Halten verschiedener langgestreckter medizinische Instrumente ausgebildet sein. Ferner kann die Haltevorrichtung als Irisblende, umfassend die zumindest drei Blendenelemente 13, ausgebildet sein. Gemäß einer weiteren Ausführungsform der vorgeschlagenen Haltevorrichtung kann das zumindest eine erste Kopplungselement 14 des jeweiligen Blendenelements 13 und/oder das zumindest eine zweite Kopplungselement 11 des ersten 1 und/oder des zweiten Aufnahmeelements 2 als Passschraube (engl. shoulder screw) ausgebildet sein. Dabei kann die mechanische Kopplung zwischen zumindest einem ersten 14 und zumindest einem zweiten Kopplungselement 11 vorteilhafterweise durch eine Zwangsführung der Passschraube in einer länglichen Führung erfolgen.

Ferner kann das Halten des medizinischen Instruments mit einer höheren Anzahl von Blendenelementen 13 gleichmäßiger erfolgen.

Vorteilhafterweise ist ein Durchmesser der Öffnung 12 des ersten 1 und des zweiten Aufnahmeelements 2 proportional zu einer Rotationsbewegung des ersten 1 Aufnahmeelements relativ zum zweiten Aufnahmeelement 2 um die gemeinsame Rotationsachse R herum.

Hierdurch können verschieden ausgebildete medizinische Instrumente, beispielsweise Herzkatheter, insbesondere verschiedener Größen nach dem French-Katheter-System, und/oder Katheter zur intrakardialen Echokardiographie (engl. intracardiac-echo, ICE) und/oder Führungsdrähte, von derselben Haltevorrichtung gehalten werden. Dabei können die medizinischen Instrumente Durchmesser im Submillimeterbereich, beispielsweise koronare Führungsdrähte mit einem Durchmesser von 0,25 mm, über den Millimeterbereich, beispielsweise ICE-Katheter mit einem Durchmesser von 3,3 mm, bis hin zum Zentimeterbereich aufweisen, beispielsweise Bronchoskope und/oder Laparoskope mit einem Durchmesser von über 14 mm. Dabei kann die Haltevorrichtung beispielsweise zum Halten einer großen Bandbreite von Durchmessern medizinischer Instrumente ausgebildet sein, beispielsweise medizinische Instrumente mit Durchmessern zwischen 0,25 mm und 14 mm. Ferner kann die Haltevorrichtung zum Halten medizinischer Instrumente einer vorbestimmten Bandbreite von Durchmessern ausgebildet sein, beispielsweise verschiedene Führungsdrähte mit Durchmessern im Submillimeterbereich und/oder verschiedene Katheter mit Durchmessern im Millimeterbereich und/oder verschiedene Endoskope mit Durchmessern im Zentimeterbereich. Hierdurch kann ein Raumbedarf, insbesondere ein räumliches Ausmaß, der Haltevorrichtung je nach Anwendung minimiert werden.

In Fig. 2 ist eine Ausführungsform des ersten 1 und/oder des zweiten Aufnahmeelements 2 schematisch dargestellt. Dabei ist die Öffnung 12 zur Aufnahme des medizinischen Instruments beispielhaft als Loch ausgebildet. Ferner kann das zumindest eine zweite Kopplungselement 11 des ersten 1 und/oder des zweiten Aufnahmeelements 2 als längliche Führung ausgebildet sein, in der das jeweils zumindest eine erste Kopplungselement 14 des jeweiligen Blendenelements 13 aufgenommen ist. Dabei können die länglichen Führungen des ersten 1 und/oder des zweiten Aufnahmeelements 2 als Aussparungen ausgebildet sein.

In Fig. 3 ist eine weitere Ausführungsform des ersten 1 und/oder des zweiten Aufnahmeelements 2 mit beispielhaft drei Blendenelementen 13 schematisch dargestellt. Dabei kann jedes der drei Blendenelemente 13 jeweils zwei erste Kopplungselemente 14 aufweisen, die als, insbesondere stiftförmige, Erhebungen ausgebildet sein. Ferner kann das erste 1 und/oder das zweite Aufnahmeelement 2 zu jedem der ersten Kopplungselemente 14 der drei Blendenelemente 13 jeweils ein zweites Kopplungselement 11 umfassen, das als längliche Führung ausgespart ist. Zudem können die drei Blendenelemente 13 entlang der Blendenschicht 15 eine kreissegmentförmige Form aufweisen. Des Weiteren können die drei Blendenelemente 13 gleichförmig um die gemeinsame Rotationsachse R herum angeordnet sein. Hierdurch kann ein besonders gleichmäßiges Halten eines in der Öffnung 12 des ersten 1 und des zweiten Aufnahmeelements 2 angeordneten medizinischen Instruments 33 ermöglicht werden.

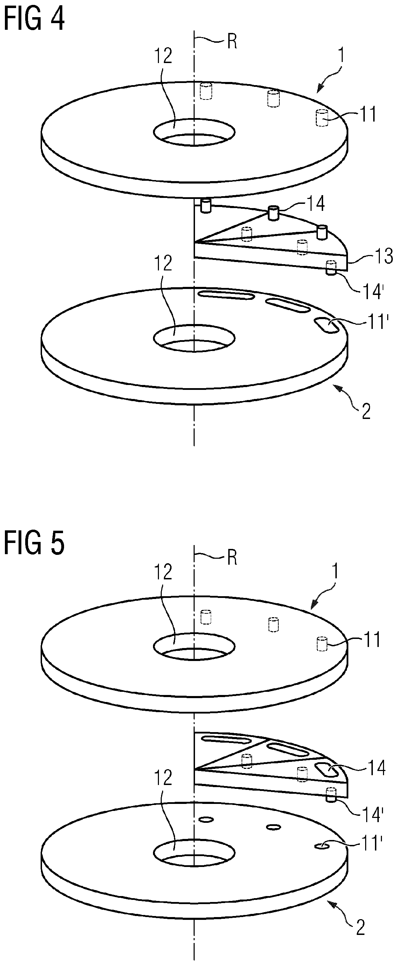

In Fig. 4 ist eine Ausführungsform der vorgeschlagenen Haltevorrichtung in einer Explosionszeichnung schematisch dargestellt. Dabei kann jedes der zumindest drei Blendenelemente 13 jeweils zwei als, insbesondere stiftförmige, Erhebung ausgebildete erste Kopplungselemente 14 und 14' umfassen. Ferner kann das erste Aufnahmeelement 1 jeweils ein als Loch ausgespartes zweites Kopplungselement 11 zur Aufnahme jeweils eines ersten Kopplungselements 14 von jeweils einem Blendenelement 13 umfassen. Hierdurch kann eine mechanische Kopplung zwischen den zumindest drei Blendenelementen 13 und dem ersten Aufnahmeelement 1 derart erreicht werden, dass jedes der zumindest drei Blendenelemente 13 innerhalb der Blendenschicht 15 drehbar gelagert ist. Ferner kann das zweite Aufnahmeelement 2 jeweils ein als längliche Führung ausgespartes zweites Kopplungselement 11` zur Aufnahme jeweils eines ersten Kopplungselements 14' von jeweils einem Blendenelement 13 umfassen. Hierdurch kann eine mechanische Kopplung zwischen den zumindest drei Blendenelementen 13 und dem zweiten Aufnahmeelement 2 derart erreicht werden, dass jedes der zumindest drei Blendenelemente innerhalb der Blendenschicht hin zur Öffnung 12 zur Aufnahme des medizinischen Instruments 33 zwangsgeführt ist.

In Fig. 5 ist eine weitere Ausführungsform der vorgeschlagenen Haltevorrichtung in einer Explosionszeichnung schematisch dargestellt. Dabei kann jedes der zumindest drei Blendenelemente 13 jeweils ein als, insbesondere stiftförmige, Erhebung ausgebildetes erstes Kopplungselement 14' auf einer dem zweiten Aufnahmeelement 2 zugewandten Seite aufweisen. Ferner kann jedes der zumindest drei Blendenelemente 13 weiterhin ein als längliche Führung ausgespartes erstes Kopplungselement 14 auf einer dem ersten Aufnahmeelement 1 zugewandten Seite aufweisen. Dabei kann das erste Aufnahmeelement 1 zu jedem der zumindest drei Blendenelemente 13 jeweils ein als stiftförmige Erhebung ausgebildetes zweites Kopplungselement 11 aufweisen, das in jeweils einem ersten Kopplungselement 14, insbesondere einer länglichen Führung, von einem Blendenelement aufgenommen ist. Ferner kann das zweite Aufnahmeelement 2 zu jedem der zumindest drei Blendenelemente 13 jeweils ein als Loch ausgespartes zweites Kopplungselement 11` aufweisen, in dem das dem zweiten Aufnahmeelement 2 zugewandte erste Kopplungselement 14' von jeweils einem Blendenelement 13 aufgenommen ist. Hierdurch kann eine mechanische Kopplung zwischen den zumindest drei Blendenelementen 13 und dem zweiten Aufnahmeelement 2 derart erreicht werden, dass jedes der zumindest drei Blendenelemente 13 innerhalb der Blendenschicht 15 drehbar gelagert ist. Ferner kann durch die mechanische Kopplung zwischen den zumindest drei Blendenelementen 13 und dem ersten Aufnahmeelement 1 eine zwangsgeführte Bewegung der zumindest drei Blendenelemente 13 innerhalb der Blendenschicht hin zur Öffnung 12 zur Aufnahme des medizinischen Instruments 33 sichergestellt werden.

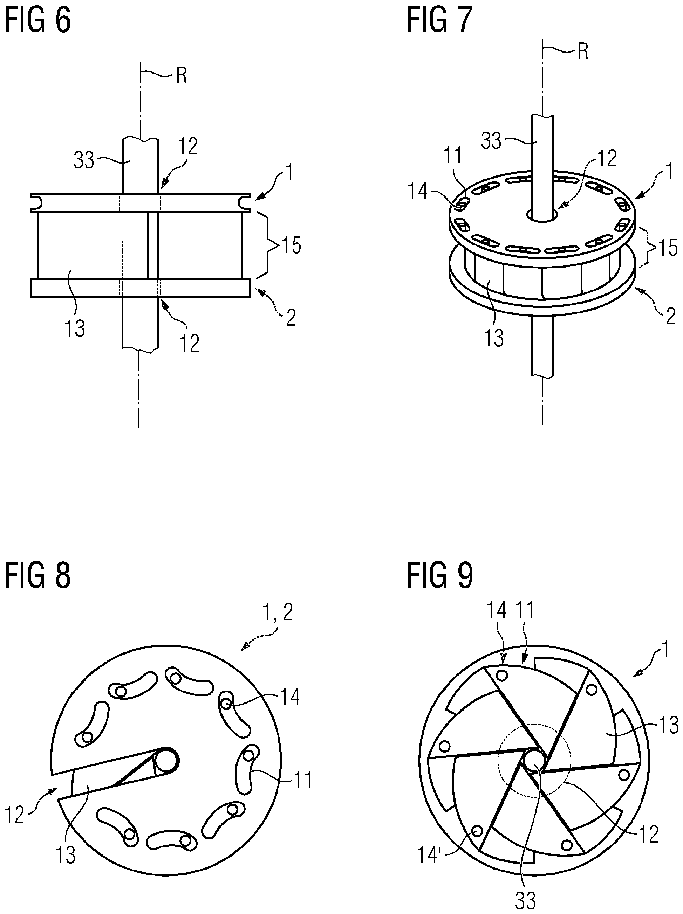

Fig. 6 zeigt schematisch eine Seitenansicht einer vorgeschlagenen Haltevorrichtung. Dabei können das erste 1 und das zweite Aufnahmeelement 2 radförmig ausgebildet und parallel zueinander angeordnet sein. Ferner kann ein medizinisches Instrument 33 parallel zur gemeinsamen Rotationsachse R innerhalb der Öffnung 12 des ersten 1 und des zweiten Aufnahmeelements 2 angeordnet sein. Dabei kann das medizinische Instrument 33 von den zumindest drei Blendenelementen 13, welche innerhalb der Blendenschicht 15 angeordnet sind, gehalten werden. Dabei kann das erste Aufnahmeelement 1 vorteilhafterweise als Riemenrad ausgebildet sein. Beispielsweise kann das erste Aufnahmeelement 1 entlang eines Umfangs zur Aufnahme eines Riemens ausgespart sein.

In Fig. 7 ist eine beispielhafte Ausführungsform der vorgeschlagenen Haltevorrichtung perspektivisch und schematisch dargestellt. Dabei kann die Öffnung 12 zur Aufnahme des medizinischen Instruments 33 als Loch ausgebildet sein. Ferner kann das medizinische Instrument 33 nach einem Schließen der Haltevorrichtung von den zumindest drei Blendenelementen 13 gehalten werden. Dabei können die zumindest drei Blendenelemente 13 ein gegenüber dem ersten 1 und/oder dem zweiten Aufnahmeelement 2 weicheres Material aufweisen. Vorteilhafterweise kann eine Grifffläche des jeweiligen Blendenelements 13 ein rutschhemmendes Material und/oder anhaftendes Material aufweisen. Hierdurch kann ein besonders sicheres Halten des medizinischen Instruments 33 durch die zumindest drei Blendenelemente 13 ermöglicht werden. Dabei können die zumindest drei Blendenelemente 13 zumindest teilweise aus einem gegenüber dem ersten 1 und/oder dem zweiten Aufnahmeelement 2 weicheren Material, beispielsweise Viskoschaum und/oder Gummi, bestehen. Zudem können die zumindest drei Blendenelemente 13 entlang der Blendenschicht 15 vorteilhafterweise eine dreieckige Form aufweisen.

In Fig. 8 ist eine weitere Ausführungsform der vorgeschlagenen Haltevorrichtung schematisch dargestellt. Dabei kann die Öffnung 12 zur Aufnahme des medizinischen Instruments 33 des ersten 1 und/oder des zweiten Aufnahmeelements 2 als Schlitz ausgebildet sein. Hierdurch kann das medizinische Instrument 33 seitlich in die Haltevorrichtung einbringbar sein. Ferner kann die Haltevorrichtung, insbesondere das erste 1 und/oder das zweite Aufnahmeelement 2, auf das medizinische Instrument 33 aufsteckbar sein. In der in Fig. 8 dargestellten Ausführungsform kann das erste 1 und/oder das zweite Aufnahmeelement 2 zu jedem der zumindest drei Blendenelemente 13 jeweils ein als längliche Führung ausgespartes zweites Kopplungselement 11 umfassen. Dabei können die zweiten Kopplungselemente 11 insbesondere gekrümmt verlaufen. Ferner kann jedes der zumindest drei Blendenelemente jeweils zumindest ein als stiftförmige Erhebung ausgebildetes erstes Kopplungselement 14 aufweisen, welches in jeweils einem der zweiten Kopplungselemente 11 des ersten 1 und/oder des zweiten Aufnahmeelements 2 aufgenommen ist. Hierdurch kann die zwangsgeführte Bewegung der zumindest drei Blendenelemente 13 hin zur Öffnung 12 zur Aufnahme des medizinischen Instruments 33, insbesondere entlang eines durch den gekrümmten Verlauf der zweiten Kopplungselemente 11 vorgegebenen Pfades, erfolgen.

Im in Fig. 9 schematisch dargestellten Ausführungsbeispiel der vorgeschlagenen Haltevorrichtung kann das erste 1 Aufnahmeelement 2 zu jedem der zumindest drei Blendenelemente 13 jeweils ein als Erhebung an einem Rand und/oder einer Seitenfläche ausgebildetes zweites Kopplungselement 14 umfassen. Dabei können die als Erhebung ausgebildeten zweiten Kopplungselemente 14 insbesondere miteinander verbunden sein und/oder die Blendenschicht 15 zumindest teilweise umschließen. Ferner können die zumindest drei Blendenelemente 13 von den als Erhebung ausgebildeten zweiten Kopplungselementen 14 zumindest teilweise umschlossen werden. Zudem kann jedes der zumindest drei Blendenelemente 13 jeweils ein erstes Kopplungselement 13 aufweisen, dass als Erhebung und/oder Aussparung an einer Seitenfläche des jeweiligen Blendenelements 13 ausgebildet ist. Dabei kann die mechanische Kopplung zwischen den zumindest drei Blendenelementen 13 und dem ersten Aufnahmeelement 1 derart zwischen den Seitenflächen der Blendenelemente 13 und den als Erhebung ausgebildeten zweiten Kopplungselementen 13 des ersten Aufnahmeelements erfolgen, dass die zumindest drei Blendenelemente hin zur Öffnung 12 zur Aufnahme des medizinischen Instruments 33 zwangsgeführt sind. Ferner kann jedes der zumindest drei Blendenelemente 13 weiterhin ein als stiftförmige Erhebung und/oder als Loch ausgespartes erstes Kopplungselement 14' zur mechanischen Kopplung an das zweite Aufnahmeelement 2 umfassen. Hierdurch kann jedes der zumindest drei Blendenelemente 13 um das erste Kopplungselement 14' herum innerhalb der Blendenschicht 15 drehbar gelagert sein.

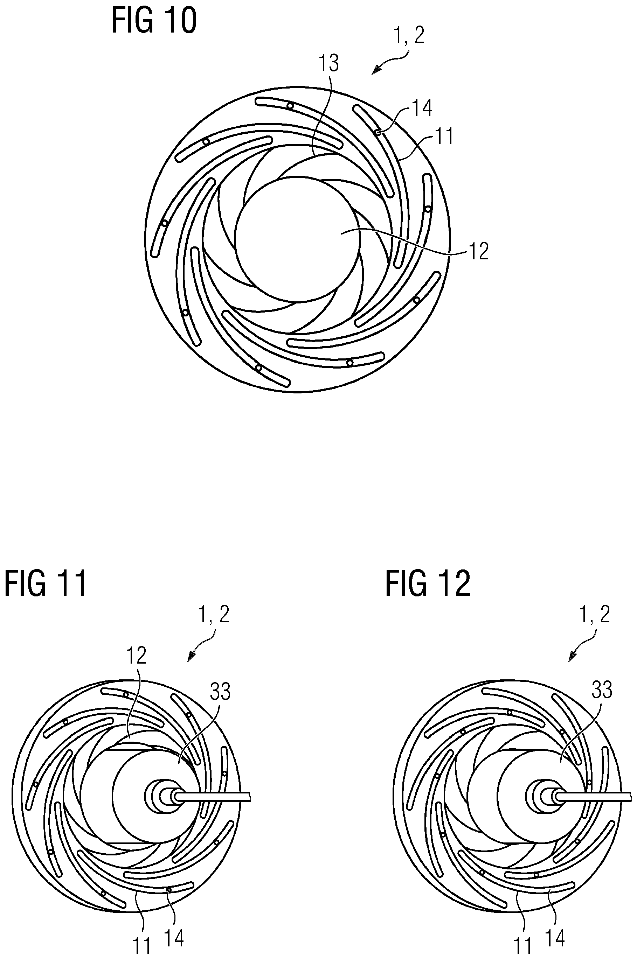

In Fig. 10 ist eine weitere Ausführungsform der vorgeschlagenen Haltevorrichtung abgebildet, wobei die zumindest drei Blendenelemente 13 innerhalb der Blendenschicht 15 zumindest teilweise überlappend angeordnet sind. Hierdurch kann eine große Anzahl von Blendenelementen 13 besonders platzsparend innerhalb der Blendenschicht 15 angeordnet werden. Ferner kann ein gleichmäßiges Halten des medizinischen Instruments 33 innerhalb der Öffnung 12 durch die zumindest drei Blendenelemente 13 ermöglicht werden.

In Fig. 11 ist eine Ausführungsform der vorgeschlagenen Haltevorrichtung schematisch dargestellt, wobei ein in der Öffnung 12 angeordnetes medizinisches Instrument 33 frei beweglich ist. Die Haltevorrichtung ist zur Aufnahme des medizinischen Instruments 33 geöffnet. Dabei sind die ersten Kopplungselemente 14 der zumindest drei Blendenelemente 13 bezüglich der zweiten Kopplungselemente 11 in einer Anfangsposition. In Fig. 12 ist die vorgeschlagene Haltevorrichtung in geschlossenem Zustand schematisch dargestellt. Dabei wird das in der Öffnung 12 angeordnete medizinische Instrument 33 von den zumindest drei Blendenelementen 13 gehalten. Durch eine Bewegung des ersten Aufnahmeelements 1 relativ zum zweiten Aufnahmeelement 2 sind die zumindest drei Blendenelemente 13 derart zwangsgeführt worden, dass diese das medizinische Instrument 33 umschließen.

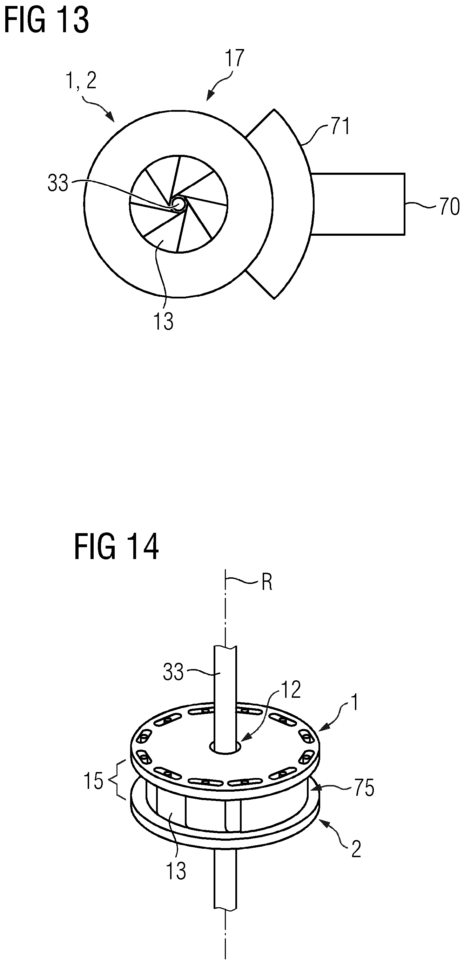

In der in Fig. 13 schematisch dargestellten Ausführungsform der vorgeschlagenen Haltevorrichtung 17, kann die Haltevorrichtung 17 weiterhin ein motorisiertes Antriebselement 70 umfassen. Dabei kann das motorisierte Antriebselement 70 zur Bewegung des ersten Aufnahmeelements 1 relativ zum zweiten Aufnahmeelement 2 um die gemeinsame Rotationsachse R herum ausgebildet sein. Dabei kann das motorisierte Antriebselement 70 einen Motor, insbesondere einen Elektromotor, umfassen. Ferner kann das motorisierte Antriebselement 70 vorteilhafterweise ein Übertragungselement 71 umfassen, welches den Motor mechanisch an das erste 1 und/oder das zweite Aufnahmeelement 2 koppelt. Dabei kann das Übertragungselement 71 beispielsweise als Riemen und/oder Zahnrad und/oder Getriebe und/oder Leitspindel ausgebildet sein.

Das medizinische Instrument 33 wird durch die zumindest drei Blendenelemente 13 solange festgehalten, wie das erste Aufnahmeelement 1 und das zweite Aufnahmeelement 2 im geschlossenen Zustand der Haltevorrichtung 17 relativ zueinander in Ruhe verharren. Dabei kann die vorgeschlagene Haltevorrichtung 17 vorteilhafterweise weiterhin ein Feststellelement umfassen, das dazu ausgebildet ist, das erste Aufnahmeelement 1 gegenüber dem zweiten Aufnahmeelement 2 zu arretieren. Insbesondere kann das erste Aufnahmeelement 1 gegenüber dem zweiten Aufnahmeelement 2 im geschlossenen Zustand der Haltevorrichtung 17 durch das Feststellelement arretiert werden. Dabei kann das Feststellelement insbesondere als Verriegelung, beispielsweise ein Hebel und/oder eine Steckverbindung zwischen dem ersten 1 und dem zweiten Aufnahmeelement 2, und/oder als Teil des motorisierten Antriebselements 70 ausgebildet sein.

In Fig. 14 ist eine weitere Ausführungsform der vorgeschlagenen Haltevorrichtung 17 schematisch dargestellt. Dabei kann die Haltevorrichtung 17 weiterhin ein Blendenfeststellelement 75 umfassen, das dazu ausgebildet ist, die zumindest drei Blendenelemente 13 zu arretieren. Dabei kann das Blendenfeststellelement 75 vorteilhafterweise innerhalb der Blendenschicht 15 und/oder um die Blendenschicht 15 herum angeordnet sein. Vorteilhafterweise kann zumindest eines der Blendenelemente 13 durch das Blendenfeststellelement 75 positionsfest arretierbar sein. Ferner kann das Blendenfeststellelement 75 dazu ausgebildet sein, das zumindest eine erste Kopplungselement 14 zumindest eines Blendenelements 13 an das korrespondierende zumindest eine zweite Kopplungselement 11 des ersten 1 und/oder des zweiten Aufnahmeelements 2 starr, insbesondere bewegungsfest, mechanisch zu koppeln.

Ferner kann das Blendenfeststellelement 75 dazu ausgebildet sein, die Anordnung der zumindest drei Blendenelemente 13, insbesondere in geschlossenem Zustand der Haltevorrichtung 17, zu arretieren. Dabei kann das Blendenfeststellelement 75 beispielsweise als umlaufendes Band und/oder Ring und/oder Hebel und/oder Schelle ausgebildet sein. Vorteilhafterweise kann die Haltevorrichtung 17 durch Lösen der Arretierung der zumindest drei Blendenelemente 13 geöffnet werden. Bei einer Anordnung des Blendenfeststellelements 13 innerhalb der Blendenschicht 15 kann eine besonders platzsparende Ausführung ermöglicht werden.

Vorteilhafterweise kann durch eine Arretierung der zumindest drei Blendenelemente 13 durch das Blendenfeststellelement 75 weiterhin eine Bewegung des ersten 1 und/oder des zweiten Aufnahmeelements 2 ermöglicht werden. Dabei kann beispielsweise das erste 1 und/oder das zweite Aufnahmeelement 2 nach einer Arretierung der zumindest drei Blendenelemente 13 durch das Blendenfeststellelement 75 aus der Haltevorrichtung 17 entfernbar sein.

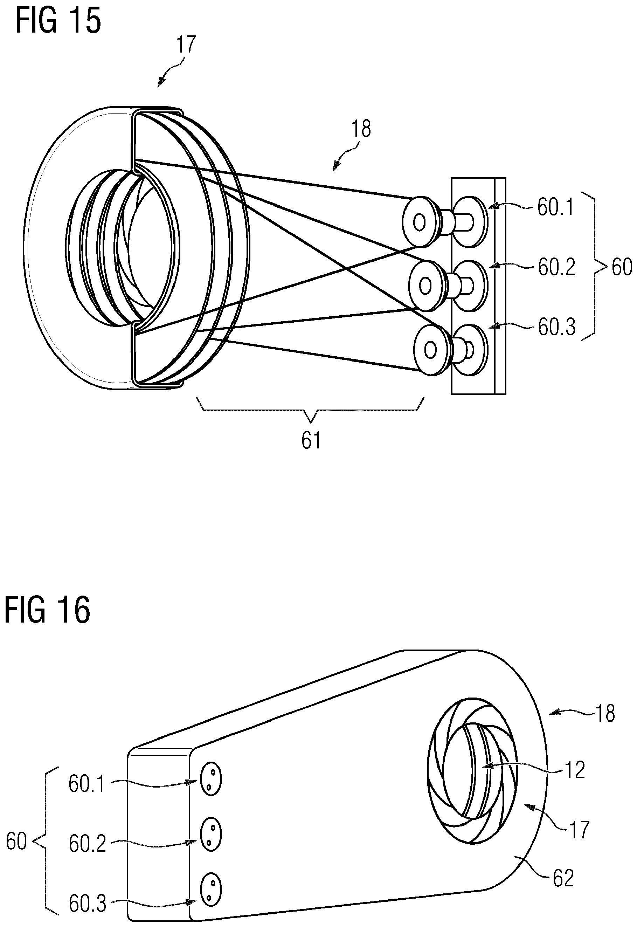

Fig. 15 zeigt eine schematische Darstellung einer vorgeschlagenen Bewegungsvorrichtung zur Bewegung eines medizinischen Instruments 33. Dabei kann die Bewegungsvorrichtung eine Haltevorrichtung 17, ein Übertragungselement 61 und ein Verbindungselement 60 umfassen. Ferner kann die Haltevorrichtung 17 zum Halten des medizinischen Instruments 33 ausgebildet sein.

Des Weiteren kann das Verbindungselement 61 eine Bewegung zwischen zumindest einem Teil der Haltevorrichtung 17 und dem Verbindungselement 60, insbesondere bidirektional übertragen. Dabei kann das Verbindungselement 60 zur Haltevorrichtung 17 beabstandet angeordnet sein.

In der in Fig. 15 schematisch dargestellte Ausführungsform der vorgeschlagenen Bewegungsvorrichtung 18 kann das Übertragungselement 61 einen Riemenantrieb aufweisen. Ferner kann das Übertragungselement 61 zusätzlich oder alternativ einen Zahnradantrieb (hier nicht gezeigt) aufweisen.

Des Weiteren kann die Haltevorrichtung 17 mehrere, insbesondere drei, Bewegungsfreiheitsgrade aufweisen. Dabei kann das Übertragungselement 61 die Bewegung gemäß der mehreren Bewegungsfreiheitsgrade zwischen der Haltevorrichtung 17 und dem Verbindungselement 60, insbesondere bidirektional und/oder gleichzeitig, überträgt. Ferner kann das Verbindungselement 60 jeweils eine Verbindungsaufnahme 60.1, 60.2 und 60.3 zu jedem der Bewegungsfreiheitsgrade der Haltevorrichtung 17 aufweisen. Dabei kann das Übertragungselement 61 beispielsweise zu jedem der drei Bewegungsfreiheitsgrade der Haltevorrichtung 17 jeweils einen Riemenantrieb zur Übertragung der Bewegung an jeweils eine der drei Verbindungsaufnahme 60.1, 60.2 und 60.3 aufweisen.

Fig. 16 zeigt eine schematische Darstellung einer vorgeschlagenen Bewegungsvorrichtung 18 mit Gehäuse 62. Dabei kann die Bewegungsvorrichtung 18 vorteilhafterweise ein Gehäuse 62 umfassen, wobei das Gehäuse 62 die Haltevorrichtung 17 und das Übertragungselement 61 derart umschließt, dass das medizinische Instrument 33 in die Haltevorrichtung 12, insbesondere die Öffnung 12 der Haltevorrichtung 12, einbringbar ist.

Vorteilhafterweise können die Haltevorrichtung 17 und das Übertragungselement 61 durch das Gehäuse 62 vor äußerer mechanischer und/oder chemischer Einwirkung geschützt werden. Ferner kann eine hygienische Reinigung der Bewegungsvorrichtung 18 durch das Gehäuse 62 vereinfacht werden. Des Weiteren kann eine mechanische Stabilität der Anordnung umfassend die Haltevorrichtung 17, das Übertragungselement 61 und das Verbindungselement 60 durch das Gehäuse 62 verbessert werden. In der in Fig. 16 dargestellten Ausführungsform umfasst das Gehäuse 62 weiterhin jeweils eine Öffnung zu jeder der Verbindungsaufnahmen 60.1, 60.2 und 60.3.

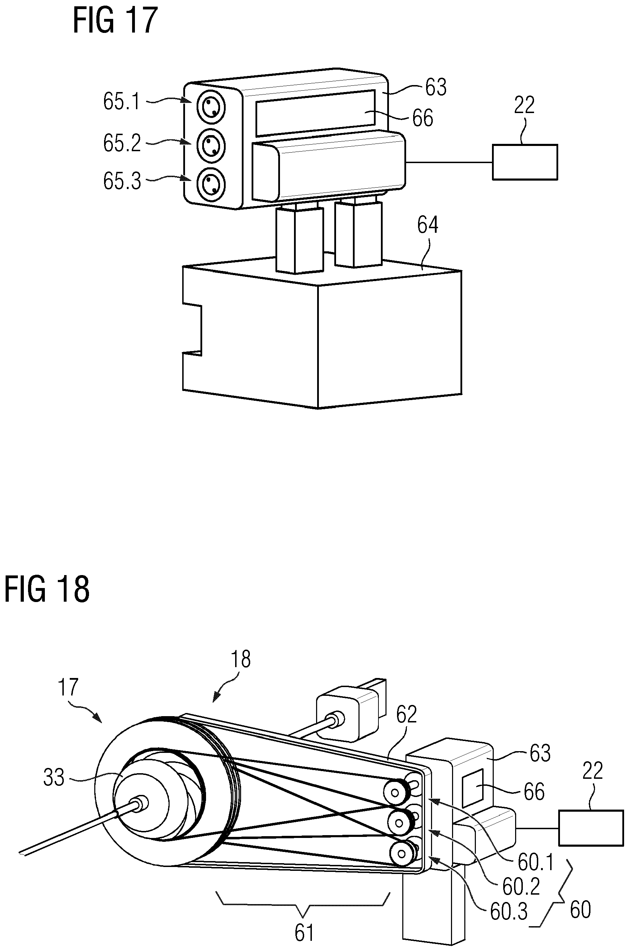

In Fig. 17 ist eine schematische Darstellung eines Motorelements 63 und eines Befestigungselements 64 dargestellt. Dabei kann eine vorgeschlagene Bewegungsvorrichtung 18 vorteilhafterweise das Motorelement 63 und das Befestigungselement 64 umfassen. Ferner kann das Befestigungselement 64 zur Befestigung der Bewegungsvorrichtung 18 an einem medizinischen Gerät und/oder einer Patientenlagerungsvorrichtung, insbesondere an einer Führungsschiene, ausgebildet sein. Zudem kann das Motorelement 63 dazu ausgebildet sein, mit dem Verbindungselement 60, insbesondere mechanisch, derart verbunden zu werden, dass das Motorelement 63 und das Verbindungselement 60 bewegungsgekoppelt sind. Hierdurch kann eine Bewegung zwischen dem Motorelement 63 und zumindest einem Teil der Haltevorrichtung 17 durch das Übertragungselement 60 übertragbar sein.

Insbesondere kann das Befestigungselement 64 eine Bewegungseinheit, beispielsweise ein Rollensystem, umfassen. Hierdurch kann eine Bewegung der Bewegungsvorrichtung 18 ermöglicht werden. Vorteilhafterweise kann die Bewegungseinheit des Befestigungselements 64 einen Motorantrieb aufweisen, wobei die Verarbeitungseinheit 22 weiterhin zur Steuerung des Motorantriebs ausgebildet sein kann.

Dabei kann das Motorelement 63 sowohl zum Öffnen und/oder Schließen der Haltevorrichtung 17, als auch zur Bewegung der Haltevorrichtung 17 als Ganzes ausgebildet sein. Hierdurch kann vorteilhafterweise eine Rotationsbewegung des in der Haltevorrichtung 17 gehaltenen medizinischen Instruments 33 ermöglicht werden. Ferner kann das Motorelement 63 auf einer Seite des ersten 1 und/oder des zweiten Aufnahmeelements 2, insbesondere entlang der gemeinsamen Rotationsachse R, angeordnet sein. Hierdurch kann eine besonders platzsparende Anordnung ermöglicht werden.

Ferner kann das Motorelement 63 ein Sensorelement 66 zur Erfassung einer Bewegung zumindest eines Teils der Haltevorrichtung 17 umfassen. Dabei kann das Sensorelement 66 beispielsweise einen optischen und/oder elektromagnetischen Sensor umfassen.

Ferner können die Haltevorrichtung 17, das Übertragungselement 61, das Verbindungselement 60, sowie das Gehäuse 62 als Einwegkassette ausgebildet sein. Dabei können das Motorelement 63 und das Befestigungselement 64 vorteilhafterweise wiederverwendbar sein, während die vorstehend beschriebene Einwegkassette mittels des Verbindungselement an das Motorelement koppelbar ist. Vorteilhafterweise kann die Einwegkassette besonders platzsparend ausgebildet sein.

Des Weiteren kann die vorgeschlagene Bewegungsvorrichtung 18 eine Verarbeitungseinheit 22 umfassen, die dazu ausgebildet ist, das Motorelement 63 und/oder die Haltevorrichtung 17 zu steuern.

In Fig. 18 ist eine schematische Darstellung einer vorgeschlagenen Bewegungsvorrichtung 18 mit Gehäuse 62 und Motorelement 63 dargestellt. Dabei kann das Motorelement 63 insbesondere mittels der drei Verbindungsaufnahmen 60.1, 60.2 und 60.3 an jeweils einen der drei Bewegungsfreiheitgrade der Haltevorrichtung 17 mechanisch gekoppelt sein. Dabei kann das Gehäuse insbesondere als sterile Barriere zwischen der Bewegungsvorrichtung 18 und dem Motorelement 63 ausgebildet sein.

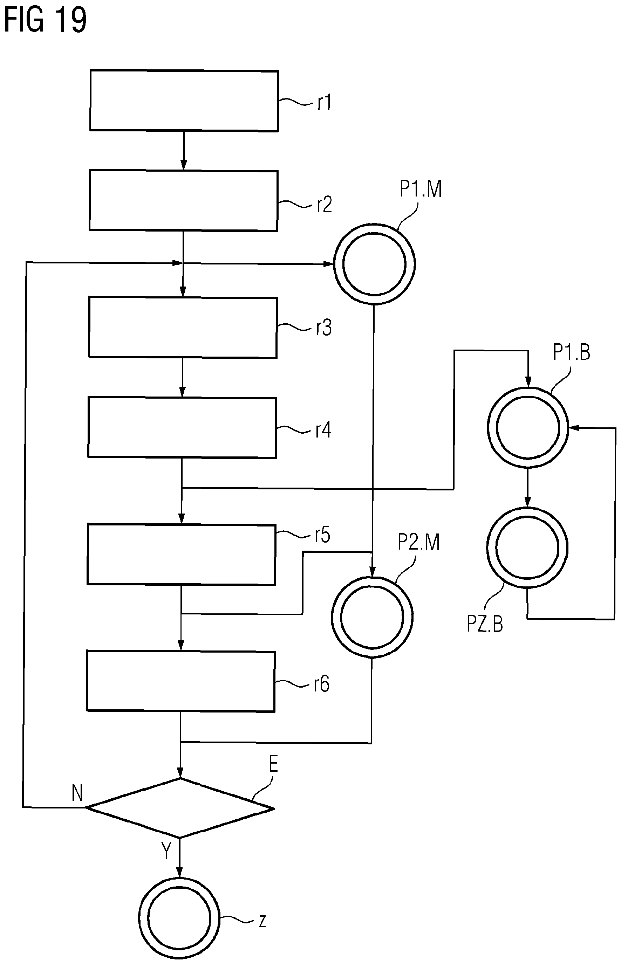

In Fig. 19 ist eine schematische Darstellung einer Ausführungsform eines Verfahrens zur Bewegung eines medizinischen Instruments mittels einer ersten Bewegungsvorrichtung 18 abgebildet. Danach kann in einem ersten Schritt r1) das medizinische Instrument 33 in der ersten Bewegungsvorrichtung 18 angeordnet werden. Ferner kann in einem Schritt r2) eine erste Position P1.M zumindest eines Abschnitts des medizinischen Instruments 33 bestimmt werden. Hiernach kann, in Schritt r3), das erste 1 und/oder das zweite Aufnahmeelement 2 der Haltevorrichtung 17 der ersten Bewegungsvorrichtung 18 derart bewegt werden, dass die zumindest drei Blendenelemente 13 das medizinische Instrument 33 halten. In einem Schritt r4) kann die erste Bewegungsvorrichtung 18 aus einer Anfangsposition Pl.B in eine Zielposition PZ.B bewegt werden, wobei das medizinische Instrument 33 zumindest abschnittsweise mitbewegt wird. In einem Schritt r5) kann eine weitere Position P2.M des zumindest einen Abschnitts des medizinischen Instruments 33 bestimmt werden. Ferner kann in einem Schritt r6) das erste 1 und/oder das zweite Aufnahmeelement 2 der Haltevorrichtung 17 der ersten Bewegungsvorrichtung 18 derart bewegt werden, dass das medizinische Instrument 33 von den zumindest drei Blendenelementen 13 freigegeben wird. Dabei können die Schritte r2) bis r6) bis zum Erreichen einer Zielposition Z des zumindest einen Abschnitts des medizinischen Instruments 33 wiederholt werden. Dabei kann insbesondere nach Schritt r6) ein Abgleich E der weiteren Position P2.M mit der Zielposition Z erfolgen.

Das hier vorgeschlagene Verfahren kann insbesondere zur automatisierten und/oder ein Bedienpersonal unterstützenden Bewegung des medizinischen Instruments 33 mittels der Bewegungsvorrichtung 18 hin zu einer Zielposition Z dienlich sein. Ferner kann das vorgeschlagene Verfahren weiterhin eine Regelung der Bewegung mittels eines Signals des Sensorelements 66 und/oder einer bildgeführten Navigation umfassen, insbesondere überwacht durch ein medizinisches Bildgebungsverfahren, beispielsweise computertomographische Angiographie und/oder Magnetresonanzangiographie und/oder ICE Ultrasonographie und/oder intravaskulären Ultraschall (IVUS) und/oder optische Kohärenztomographie (engl. optical coherence tomography, OCT) .

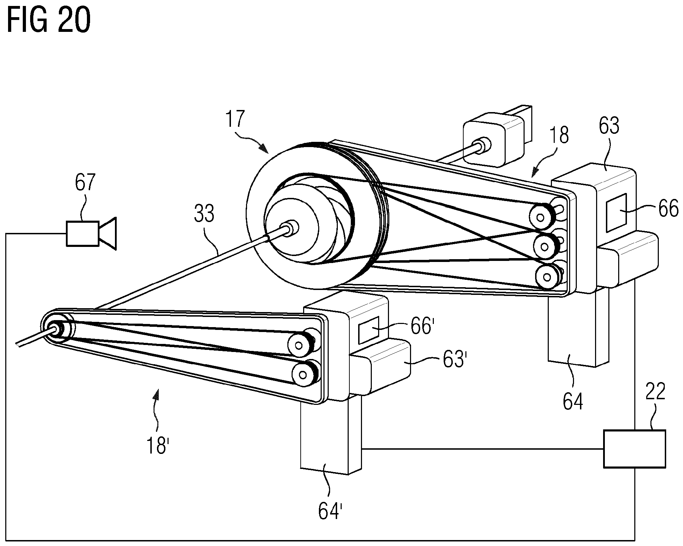

In Fig. 20 ist eine Ausführungsform einer vorgeschlagenen Koordinationsvorrichtung zur koordinierten Bewegung eines medizinischen Instruments 33 schematisch dargestellt. Dabei kann die Koordinationsvorrichtung eine erste Bewegungsvorrichtung 18 und zumindest eine weitere Bewegungsvorrichtung 18' umfassen. Ferner können die erste 18 und die zumindest eine weitere Bewegungsvorrichtung 18` beabstandet voneinander angeordnet sein. Dabei können die erste 18 und die zumindest eine weitere Bewegungsvorrichtung 18` zur Bewegung desselben medizinischen Instruments 33 ausgebildet sein. Ferner können die erste 18 und die zumindest eine weitere Bewegungsvorrichtung 18' das medizinische Instrument 33 koordiniert bewegen. Dabei kann die Koordinationsvorrichtung vorteilhafterweise eine Verarbeitungseinheit 22 zur Koordination der Bewegung der ersten 18 und der zumindest einen weiteren Bewegungsvorrichtung 18' umfassen.

Ferner kann die Koordination der Bewegung des medizinischen Instruments 33 durch die erste 18 und/oder die zumindest eine weitere Bewegungsvorrichtung 18` vorteilhafterweise einen Ausgleich einer Torsion und/oder Verformung des medizinischen Instruments 33 umfassen.

Des Weiteren kann die Koordinationsvorrichtung weiterhin eine Instrumentenerfassungseinheit 67 umfassen, die zur Identifikation des in der ersten 18 und der zumindest einen weiteren Bewegungsvorrichtung 18` aufgenommenen medizinischen Instruments 33 ausgebildet ist. Dabei kann die Bewegung des medizinischen Instruments 33 durch die erste 18 und/oder die zumindest eine weitere Bewegungsvorrichtung 18' in Abhängigkeit einer Materialeigenschaft des identifizierten medizinischen Instruments 33 erfolgen. Hierfür kann die Instrumentenerfassungseinheit 67 beispielsweise ein Signal an die Verarbeitungseinheit 22 senden.

Zudem kann die Koordinationsvorrichtung eine Zustandserfassungseinheit umfassen, die zur Erfassung einer Verformung und/oder Torsion des medizinischen Instruments 33 ausgebildet ist. Dabei kann die Zustandserfassungseinheit vorteilhafterweise jeweils ein Sensorelement 66 zur ersten Bewegungsvorrichtung 18 und zumindest ein weiteres Sensorelement 66` zur zumindest einen weitere Bewegungsvorrichtung 18` umfassen. Ferner kann die Bewegung des medizinischen Instruments 33 durch die erste 18 und/oder die zumindest eine weitere Bewegungsvorrichtung 18' vorteilhafterweise in Abhängigkeit der erfassten Verformung und/oder Torsion erfolgen. Hierfür können die Sensorelemente 66 und 66` der Zustandserfassungseinheit vorteilhafterweise jeweils ein Signal an die Verarbeitungseinheit 22 senden.

Des Weiteren können die erste 18 und die zumindest eine weitere Bewegungsvorrichtung 18` mittels jeweils einem Befestigungselement 64 und 64` entlang zumindest einer gemeinsamen Bewegungsachse, insbesondere konstant zueinander beabstandet, beweglich angeordnet sein.

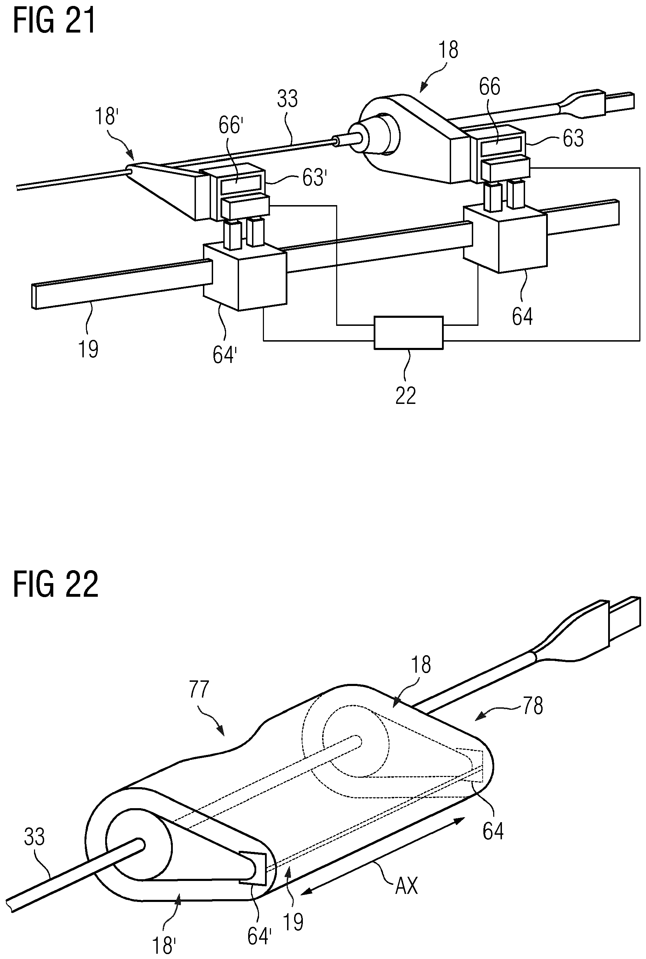

In Fig. 21 ist eine weitere Ausführungsform der vorgeschlagenen Koordinationsvorrichtung schematisch dargestellt. Dabei können die erste 18 und die zumindest eine weitere Bewegungsvorrichtung 18' mittels des jeweiligen Befestigungselements 64 und 64` an einer gemeinsamen Schiene 19 beweglich angeordnet sein. Insbesondere können die Befestigungselemente 64 und 64` jeweils eine Bewegungseinheit, beispielsweise ein Rollensystem, umfassen. Hierdurch kann eine entlang der gemeinsamen Schiene 19 geführte Bewegung der ersten 18 und der zumindest einen weiteren Bewegungsvorrichtung 18` ermöglicht werden. Vorteilhafterweise kann zumindest eine der Bewegungseinheiten der Befestigungselemente 64 und 64` einen Motorantrieb aufweisen, wobei die Verarbeitungseinheit 22 weiterhin zur, insbesondere koordinierten, Steuerung des Motorantriebs ausgebildet sein kann. Dabei kann insbesondere eine Bewegungseinheit der Befestigungselemente 64 und/oder 64` passiv bewegbar sein. Ferner können die erste 18 und die zumindest eine weitere Bewegungsvorrichtung 18', insbesondere während einer Intervention, durch sterile Hüllen, insbesondere Gehäuse, umschließbar sein.

In Fig. 22 ist eine weitere Ausführungsform der vorgeschlagenen Koordinationsvorrichtung schematisch dargestellt. Dabei kann die Koordinationsvorrichtung weiterhin ein Griffelement 77 umfassen, wobei die Anordnung der ersten 18 und der zumindest einen weiteren Bewegungsvorrichtung 18' entlang der zumindest einen gemeinsamen Bewegungsachse AX beweglich an dem Griffelement befestigt sind. Dabei kann das Griffelement 77 von einem Bedienpersonal haltbar sein. Vorteilhafterweise kann die Anordnung der ersten 18 und der zumindest einen weiteren Bewegungsvorrichtung 18` von einem Gehäuse 78 umschlossen sein. Dabei kann das Gehäuse 78 ferner das Griffelement 77 umfassen und/oder als Griffelement 77 ausgebildet sein.

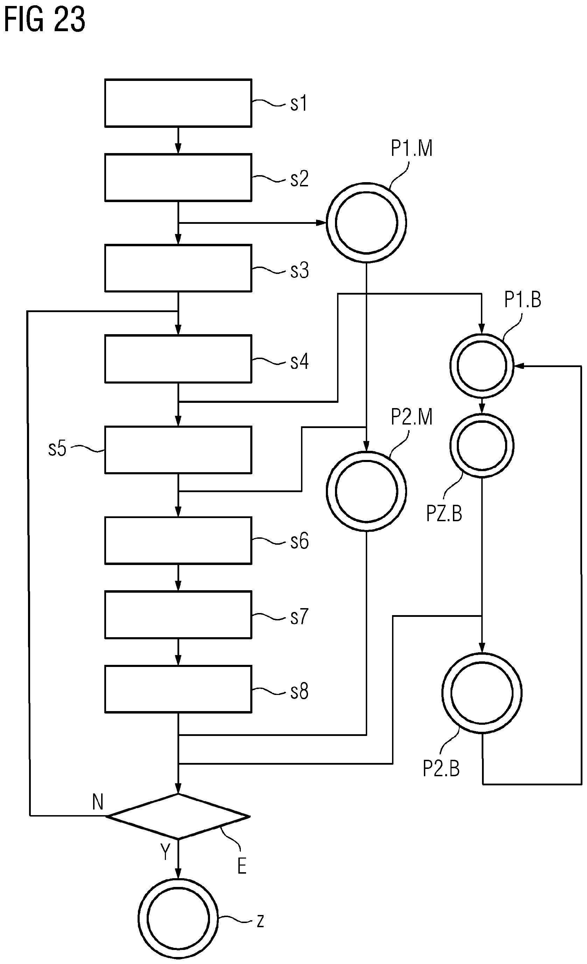

In Fig. 23 ist ein Ablaufdiagramm einer Ausführungsform eines Verfahrens zur Koordination einer Bewegung eines medizinischen Instruments 33 mittels einer Koordinationsvorrichtung schematisch dargestellt. Dabei kann in einem ersten Schritt s1) das medizinische Instrument 33 in der ersten 18 und der zumindest einen weiteren Bewegungsvorrichtung 18` angeordnet werden. Des Weiteren kann in einem Schritt s2) eine erste Position P1.M zumindest eines Abschnitts des medizinischen Instruments 33 bestimmt werden. Hiernach kann in einem Schritt s3) das erste 1 und/oder das zweite Aufnahmeelement 2 der Haltevorrichtung 17 der ersten Bewegungsvorrichtung 18 derart bewegt werden, dass die jeweils zumindest drei Blendenelemente 13 das medizinische Instrument 33 halten. Ferner kann in einem Schritt s4) die erste Bewegungsvorrichtung 18 aus einer Anfangsposition Pl.B in eine Zielposition PZ.B bewegt werden. Dabei kann das medizinische Instrument 33 zumindest abschnittsweise mitbewegt werden. In einem Schritt s5) kann eine weitere Position P2.M des zumindest einen Abschnitts des medizinischen Instruments 33 bestimmt werden. In einem Schritt s6) kann das erste 1 und/oder das zweite Aufnahmeelement 2 der Haltevorrichtung 17 der zumindest einen weiteren Bewegungsvorrichtung 18` derart bewegt werden, dass die jeweils zumindest drei Blendenelemente 13 das medizinische Instrument 33 halten. Hiernach kann in einem Schritt s7) das erste 1 und/oder das zweite Aufnahmeelement der Haltevorrichtung 17 der ersten Bewegungsvorrichtung 18 derart bewegt werden, dass das medizinische Instrument 33 durch die zumindest drei Blendenelemente 13 freigegeben wird. In einem Schritt s8) kann die erste Bewegungsvorrichtung 18 aus der Zielposition PZ.B in eine weitere Anfangsposition P2.B bewegt werden. Dabei kann die weitere Anfangsposition P2.B in Schritt s4) als Anfangsposition Pl.B vorgegeben werden. Ferner können die Schritte s1) bis s8) bis zum Erreichen einer Zielposition Z des zumindest einen Abschnitts des medizinischen Instruments 33 wiederholt werden. Dabei kann insbesondere nach Schritt s8) ein Abgleich E der weiteren Position P2.M mit der Zielposition Z erfolgen.

Hierdurch kann eine nahezu unendliche Bewegung des medizinischen Instruments 33 ermöglicht werden. Ferner kann eine von einem Abstand der ersten 18 gegenüber der zumindest einen weiteren Bewegungsvorrichtung 18` unabhängige Bewegungstrajektorie des medizinischen Instruments durch wiederholte Ausführung der Schritte s1) bis s8) realisiert werden.

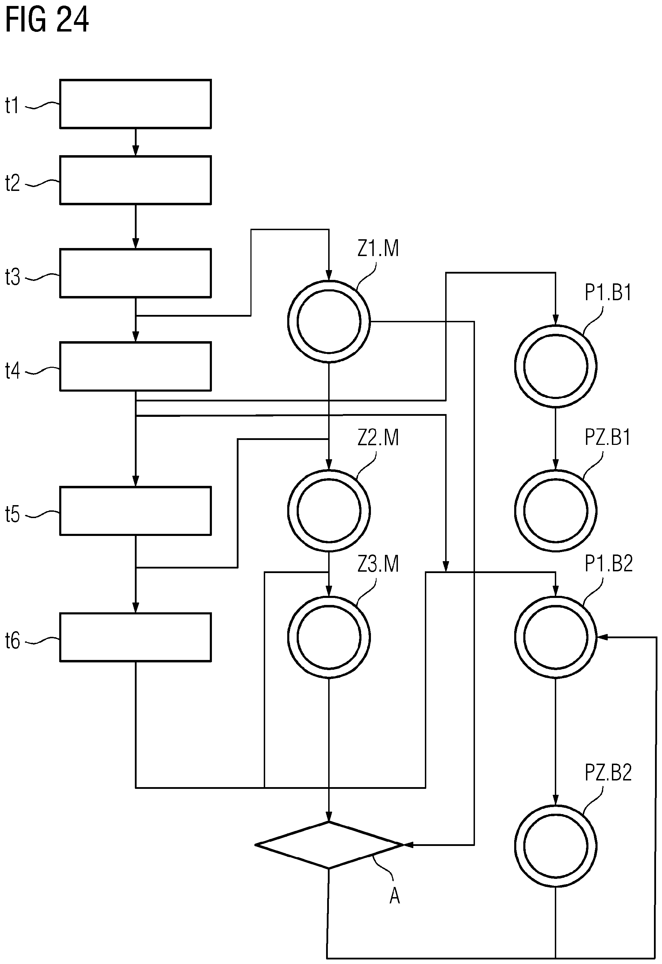

In Fig. 24 ist ein Ablaufdiagramm einer Ausführungsform eines Verfahrens zur optimierten Bewegung eines medizinischen Instruments 33 mittels einer Koordinationsvorrichtung schematisch dargestellt. Dabei kann die Koordinationsvorrichtung vorteilhafterweise eine Zustandserfassungseinheit zur Erfassung eines Zustands einer Torsion und/oder Verformung des medizinischen Instruments 33 umfassen. In einem ersten Schritt t1) kann das medizinische Instrument 33 in der ersten 18 und der zumindest einen weiteren Bewegungsvorrichtung 18` angeordnet werden. Hiernach kann in einem Schritt s2) das erste 1 und/oder das zweite Aufnahmeelement 2 der Haltevorrichtung 17 der ersten 18 und der zumindest einen weiteren Bewegungsvorrichtung 18` derart bewegt werden, dass die jeweils zumindest drei Blendenelemente 13 das medizinische Instrument 33 halten. In einem Schritt s3) kann ein Anfangszustand Z1.M einer Torsion und/oder Verformung des medizinischen Instruments 33 mittels der Zustandserfassungseinheit erfasst werden. Hiernach kann in einem Schritt t4) eine Bewegung der ersten Bewegungsvorrichtung 18 aus einer Anfangsposition P1.B1 in eine Zielposition PZ.B1 erfolgen, wobei das medizinische Instrument 33 zumindest abschnittsweise mitbewegt wird. In einem Schritt t5) kann ein Zwischenzustand Z2.M einer Torsion und/oder Verformung des medizinischen Instruments 33 mittels der Zustandserfassungseinheit erfasst werden. Hiernach kann in Schritt t6) die zumindest eine weitere Bewegungsvorrichtung 18` in Abhängigkeit zur Bewegung der ersten Bewegungsvorrichtung in Schritt t4) und/oder zum erfassten Zwischenzustand Z2.M bewegt werden. Dabei kann die zumindest eine weitere Bewegungsvorrichtung 18` aus einer Anfangsposition P1.B2 in eine Zielposition P2.B2 bewegt werden. Ferner kann in Schritt t7), insbesondere nach und/oder während der Bewegung der zumindest einen weiteren Bewegungsvorrichtung 18` in Schritt t6), ein weiterer Zwischenzustand Z3.M des zumindest einen Abschnitts des medizinischen Instruments 33 mittels der Zustandserfassungseinheit erfasst werden. Dabei kann vorteilhafterweise eine Abweichung A zwischen dem weiteren Zwischenzustand Z3.M und dem Anfangszustand Z1,M durch die Bewegung in Schritt t6) minimiert werden.

Die in den beschriebenen Figuren enthaltenen schematischen Darstellungen bilden keinerlei Maßstab oder Größenverhältnis ab.

Es wird abschließend noch einmal darauf hingewiesen, dass es sich bei den vorhergehend detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden Teil-Komponenten bestehen, die gegebenenfalls auch räumlich verteilt sein können.

## Patentansprüche

1. Haltevorrichtung (17) zum Halten eines medizinischen Instruments (33) umfassend ein erstes Aufnahmeelement (1), ein zweites Aufnahmeelement (2) und zumindest drei Blendenelemente (13),
wobei die zumindest drei Blendenelemente (13) innerhalb einer Blendenschicht (15) zwischen dem ersten (1) und dem zweiten Aufnahmeelement (2) um eine gemeinsame Rotationsachse (R) des ersten (1) und des zweiten Aufnahmeelements (2) herum angeordnet sind,
wobei die gemeinsame Rotationsachse (R) im Wesentlichen senkrecht zur Blendenschicht (15) verläuft,
wobei das erste (1) und das zweite Aufnahmeelement (2) jeweils eine Öffnung (12) zur Aufnahme des medizinischen Instruments (33) aufweisen,
wobei das erste (1) und das zweite Aufnahmeelement (2) um die gemeinsame Rotationsachse (R) herum relativ zueinander beweglich sind,
wobei die zumindest drei Blendenelemente (13) jeweils zumindest ein erstes Kopplungselement (14) umfassen,
wobei das erste (1) und/oder das zweite Aufnahmeelement (2) zu jedem der zumindest drei Blendenelemente (13) jeweils zumindest ein zweites Kopplungselement (11) umfasst,
wobei jedes der zumindest drei Blendenelemente (13) innerhalb der Blendenschicht (15), in der diese angeordnet sind, durch mechanische Kopplung zwischen dem jeweiligen zumindest einen ersten Kopplungselement (14) des Blendenelements (13) und dem zumindest einen zweiten Kopplungselement (11) zwangsgeführt ist,
wobei bei einer Bewegung des ersten Aufnahmeelements (1) relativ zum zweiten Aufnahmeelement (2) um die gemeinsame Rotationsachse (R) herum eine durch das erste (1) und das zweite Aufnahmeelement (2) derart zwangsgeführte Bewegung der zumindest drei Blendenelemente (13) innerhalb der Blendenschicht (15) hin zur Öffnung (12) zur Aufnahme des medizinischen Instruments (33) erfolgt,
dass die zumindest drei Blendenelemente (13) ein in der Öffnung (12) des ersten (1) und des zweiten Aufnahmeelements (2) angeordnetes medizinisches Instrument (33) halten,
wobei die zumindest drei Blendenelemente (13) ein gegenüber dem ersten (1) und/oder dem zweiten Aufnahmeelement (2) weicheres Material aufweisen.

2. Haltevorrichtung (17) nach Anspruch 1, **dadurch gekennzeichnet, dass** das zumindest eine erste Kopplungselement (14) des jeweiligen Blendenelements (13) als längliche Führung ausgebildet ist, in der jeweils zumindest ein zweites Kopplungselement (11) des ersten (1) und/oder des zweiten Aufnahmeelements (2) aufgenommen ist.

3. Haltevorrichtung (17) nach Anspruch 1 oder 2, **dadurch gekennzeichnet, dass** das zumindest eine zweite Kopplungselement (11) des ersten (1) und/oder des zweiten Aufnahmeelements (2) als längliche Führung ausgebildet ist, in der das jeweils zumindest eine erste Kopplungselement (14) des jeweiligen Blendenelements (13) aufgenommen ist.

4. Haltevorrichtung (17) nach einem der vorangehenden Ansprüche, **dadurch gekennzeichnet, dass** die zumindest drei Blendenelemente (13) innerhalb der Blendenschicht (15) zumindest teilweise gegenseitig überlappend angeordnet sind.

5. Haltevorrichtung (17) nach einem der vorangehenden Ansprüche, **dadurch gekennzeichnet, dass** das jeweils zumindest eine erste Kopplungselement (14) des jeweiligen Blendenelements (13) und/oder das zumindest eine zweite Kopplungselement (11) des ersten (1) und/oder des zweiten Aufnahmeelements (2) als Erhebung und/oder Aussparung ausgebildet ist.

6. Haltevorrichtung (17) nach einem der vorangehenden Ansprüche, **dadurch gekennzeichnet, dass** die zumindest drei Blendenelemente (13) gleichförmig um die gemeinsame Rotationsachse (R) herum angeordnet sind.

7. Haltevorrichtung (17) nach einem der vorangehenden Ansprüche, **dadurch gekennzeichnet, dass** die zumindest drei Blendenelemente (13) entlang der Blendenschicht (15) eine dreieckige und/oder kreissegmentförmige Form aufweisen.

8. Haltevorrichtung (17) nach einem der vorangehenden Ansprüche, **dadurch gekennzeichnet, dass** die Öffnung (12) zur Aufnahme des medizinischen Instruments (33) des ersten (1) und/oder des zweiten Aufnahmeelements (2) als Loch und/oder Schlitz ausgebildet ist.

9. Haltevorrichtung nach einem der vorangehenden Ansprüche, **dadurch gekennzeichnet, dass** das erste und/oder das zweite Aufnahmeelement radförmig ausgebildet ist.

10. Haltevorrichtung (17) nach Anspruch 9, **dadurch gekennzeichnet, dass** das erste (1) und/oder das zweite Aufnahmeelement (2) als Zahnrad und/oder Riemenrad ausgebildet ist.

11. Haltevorrichtung (17) nach einem der vorangehenden Ansprüche weiterhin umfassend ein motorisiertes Antriebselement (70), das zur Bewegung des ersten Aufnahmeelements (1) relativ zum zweiten Aufnahmeelement (2) um die gemeinsame Rotationsachse (R) herum ausgebildet ist.

12. Haltevorrichtung (17) nach einem der vorangehenden Ansprüche, weiterhin umfassend ein Feststellelement, das dazu ausgebildet ist, das erste Aufnahmeelement (1) gegenüber dem zweiten Aufnahmeelement (2) zu arretieren.

13. Haltevorrichtung (17) nach einem der vorangehenden Ansprüche, weiterhin umfassend ein Blendenfeststellelement (75), das dazu ausgebildet ist, die zumindest drei Blendenelemente (13) zu arretieren.

14. Haltevorrichtung (17) nach einem der vorangehenden Ansprüche, wobei das erste Aufnahmeelement (1) zu jedem der zumindest drei Blendenelemente (13) jeweils ein zweites Kopplungselement (11) umfasst, das als längliche Führung ausgespart ist und geradlinig verläuft,
wobei das zweite Aufnahmeelement (2) zu jedem der zumindest drei Blendenelemente (13) jeweils ein zweites Kopplungselement (11) umfasst, das als längliche Führung ausgespart ist und gekrümmt verläuft,
wobei das erste (1) und das zweite Aufnahmeelement (2) radförmig ausgebildet sind,
wobei das erste (1) und/oder das zweite Aufnahmeelement (2) als Zahnrad ausgebildet ist,
wobei die Öffnung (12) zur Aufnahme des medizinischen Instruments (33) des ersten (1) und des zweiten Aufnahmeelements (2) als Schlitz ausgebildet ist und von der gemeinsamen Rotationsachse (R) begrenzt wird,
wobei die zumindest drei Blendenelemente (13) eine dreieckige Form aufweisen,
wobei jedes der zumindest drei Blendenelemente (13) jeweils zwei erste Kopplungselemente (14) aufweist, die als stiftförmige Erhebungen ausgebildet sind,
wobei jeweils ein erstes der zwei ersten Kopplungselemente (14) von jedem der zumindest drei Blendenelemente (13) auf einer dem ersten Aufnahmeelement (1) zugewandten Seite des Blendenelements (13) angeordnet ist,
wobei jeweils ein zweites der zwei ersten Kopplungselemente (14) von jedem der zumindest drei Blendenelemente (13) auf einer dem zweiten Aufnahmeelement (2) zugewandten Seite des Blendenelements (13) angeordnet ist,
wobei die zwei ersten Kopplungselemente (14) von jedem der zumindest drei Blendenelemente (13) nicht auf einer zur gemeinsamen Rotationsachse (R) parallelen Raumachse liegen.

## Claims

1. Holding device (17) for holding a medical instrument (33), the holding device (17) comprising a first receiving element (1), a second receiving element (2) and at least three diaphragm elements (13),
wherein the at least three diaphragm elements (13) are arranged, about a common axis of rotation (R) of the first receiving element (1) and of the second receiving element (2), within a diaphragm layer (15) between the first receiving element (1) and the second receiving element (2),
wherein the common axis of rotation (R) runs substantially at right angles to the diaphragm layer (15),
wherein the first receiving element (1) and the second receiving element (2) each have an opening (12) for receiving the medical instrument (33),
wherein the first receiving element (1) and the second receiving element (2) are movable relative to each other about the common axis of rotation (R),
wherein the at least three diaphragm elements (13) each comprise at least one first coupling element (14),
wherein the first receiving element (1) and/or the second receiving element (2) in each case comprises, for each of the at least three diaphragm elements (13), at least one second coupling element (11),
wherein each of the at least three diaphragm elements (13) within the diaphragm layer (15) in which they are arranged is positively guided by mechanical coupling between the respective at least one first coupling element (14) of the diaphragm element (13) and the at least one second coupling element (11),
wherein, upon a movement of the first receiving element (1) relative to the second receiving element (2) about the common axis of rotation (R), the at least three diaphragm elements (13) within the diaphragm layer (15) are positively guided by the first receiving element (1) and the second receiving element (2) towards the opening (12) for receiving the medical instrument (33), such that the at least three diaphragm elements (13) hold a medical instrument (33) arranged in the opening (12) of the first receiving element (1) and of the second receiving element (2), wherein the at least three diaphragm elements (13) have a softer material compared to the first receiving element (1) and/or the second receiving element (2).

2. Holding device (17) according to Claim 1, **characterized in that** the at least one first coupling element (14) of the respective diaphragm element (13) is configured as an elongate guide in which in each case at least one second coupling element (11) of the first receiving element (1) and/or of the second receiving element (2) is received.

3. Holding device (17) according to Claim 1 or 2, **characterized in that** the at least one second coupling element (11) of the first receiving element (1) and/or of the second receiving element (2) is configured as an elongate guide in which the respective at least one first coupling element (14) of the respective diaphragm element (13) is received.

4. Holding device (17) according to one of the preceding claims, **characterized in that** the at least three diaphragm elements (13) within the diaphragm layer (15) are arranged overlapping one another at least in part.

5. Holding device (17) according to one of the preceding claims, **characterized in that** the at least one first coupling element (14) of the respective diaphragm element (13) and/or the at least one second coupling element (11) of the first receiving element (1) and/or of the second receiving element (2) is in each case configured as an elevation and/or cutout.

6. Holding device (17) according to one of the preceding claims, **characterized in that** the at least three diaphragm elements (13) are arranged uniformly about the common axis of rotation (R).

7. Holding device (17) according to one of the preceding claims, **characterized in that** the at least three diaphragm elements (13) along the diaphragm layer (15) have the shape of a triangle and/or a segment of a circle.

8. Holding device (17) according to one of the preceding claims, **characterized in that** the opening (12), receiving the medical instrument (33), of the first receiving element (1) and/or of the second receiving element (2) is configured as a hole and/or slot.

9. Holding device according to one of the preceding claims, **characterized in that** the first receiving element and/or the second receiving element is configured in the shape of a wheel.

10. Holding device (17) according to Claim 9, **characterized in that** the first receiving element (1) and/or the second receiving element (2) is configured as a toothed wheel and/or a belt wheel.

11. Holding device (17) according to one of the preceding claims, further comprising a motorized drive element (70) configured to move the first receiving element (1) relative to the second receiving element (2) about the common axis of rotation (R).

12. Holding device (17) according to one of the preceding claims, further comprising a securing element configured to lock the first receiving element (1) relative to the second receiving element (2).

13. Holding device (17) according to one of the preceding claims, further comprising a diaphragm -securing element (75) configured to lock the at least three diaphragm elements (13).

14. Holding device (17) according to one of the preceding claims, wherein the first receiving element (1) comprises, for each of the at least three diaphragm elements (13), in each case a second coupling element (11) which is cut out as an elongate guide and runs in a straight line,
wherein the second receiving element (2) comprises, for each of the at least three diaphragm elements (13), in each case a second coupling element (11) which is cut out as an elongate guide and runs in a curve,
wherein the first receiving element (1) and the second receiving element (2) are configured in the shape of a wheel,
wherein the first receiving element (1) and/or the second receiving element (2) is configured as a toothed wheel,
wherein the opening (12), receiving the medical instrument (33), of the first receiving element (1) and of the second receiving element (2) is configured as a slot and is delimited by the common axis of rotation (R),
wherein the at least three diaphragm elements (13) have a triangular shape,
wherein each of the at least three diaphragm elements (13) has two first coupling elements (14), which are configured as pin-shaped elevations,
wherein a first of the two first coupling elements (14) of each of the at least three diaphragm elements (13) is arranged on a side of the diaphragm element (13) facing towards the first receiving element (1),
wherein a second of the two first coupling elements (14) of each of the at least three diaphragm elements (13) is arranged on a side of the diaphragm element (13) facing towards the second receiving element (2),
wherein the two first coupling elements (14) of each of the at least three diaphragm elements (13) do not lie on a spatial axis parallel to the common axis of rotation (R).

## Revendications

1. Dispositif de retenue (17) destiné à maintenir un instrument médical (33) comprenant un premier élément de réception (1), un deuxième élément de réception (2) et au moins trois éléments obturateurs (13),
les au moins trois éléments obturateurs (13) étant disposés à l'intérieur d'une couche d'obturation (15) entre le premier (1) et le deuxième élément de réception (2) autour d'un axe de rotation commun (R) du premier (1) et du deuxième élément de réception (2),
l'axe de rotation commun (R) s'étendant sensiblement perpendiculairement à la couche d'obturation (15),
le premier (1) et le deuxième élément de réception (2) comportant chacun une ouverture (12) destinée à recevoir l'instrument médical (33),
le premier (1) et le deuxième élément de réception (2) étant mobiles l'un par rapport à l'autre autour de l'axe de rotation commun (R),
les au moins trois éléments obturateurs (13) comprenant chacun au moins un premier élément d'accouplement (14),
le premier (1) et/ou le deuxième élément de réception (2) comprenant au moins un deuxième élément d'accouplement (11) pour chacun des au moins trois éléments obturateurs(13),
chacun des au moins trois éléments obturateurs (13) étant soumis à un guidage forcé à l'intérieur de la couche d'obturation (15), dans laquelle ils sont disposés, par accouplement mécanique entre l'au moins un premier élément d'accouplement respectif (14) de l'élément obturateur (13) et l'au moins un deuxième élément d'accouplement (11),
lors d'un mouvement du premier élément de réception (1) par rapport au deuxième élément de réception (2) autour de l'axe de rotation commun (R), un mouvement des au moins trois éléments obturateurs (13), qui est soumis un guidage forcé par le premier (1) et le deuxième élément de réception (2), étant effectué à l'intérieur de la couche d'obturation (15) en direction de l'ouverture (12) afin de recevoir l'instrument médical (33), manière à ce que les au moins trois éléments obturateurs (13) maintiennent un instrument médical (33) disposé dans l'ouverture (12) du premier (1) et du deuxième élément de réception (2),
les au moins trois éléments obturateurs (13) comportant un matériau plus mou que celui du premier (1) et/ou du deuxième élément de réception (2).

2. Dispositif de retenue (17) selon la revendication 1, **caractérisé en ce que** l'au moins un premier élément d'accouplement (14) de l'élément obturateur respectif (13) est conçu comme un guide allongé dans lequel est reçu au moins un deuxième élément d'accouplement (11) du premier (1) et/ou du deuxième élément de réception (2).

3. Dispositif de retenue (17) selon la revendication 1 ou 2, **caractérisé en ce que** l'au moins un deuxième élément d'accouplement (11) du premier (1) et/ou du deuxième élément de réception (2) est conçu comme un guide allongé dans lequel est reçu l'au moins un premier élément d'accouplement (14) de l'élément obturateur respectif (13).

4. Dispositif de retenue (17) selon l'une des revendications précédentes, **caractérisé en ce que** les au moins trois éléments obturateurs (13) sont disposés à l'intérieur de la couche d'obturation (15) en se recouvrant au moins partiellement.

5. Dispositif de retenue (17) selon l'une des revendications précédentes, **caractérisé en ce que** l'au moins un premier élément d'accouplement (14) de l'élément obturateur respectif (13) et/ou l'au moins un deuxième élément d'accouplement (11) du premier (1) et/ou du deuxième élément de réception (2) sont conçus sous la forme d'une surélévation et/ou d'un évidement.

6. Dispositif de retenue (17) selon l'une des revendications précédentes, **caractérisé en ce que** les au moins trois éléments obturateurs (13) sont disposés uniformément autour de l'axe de rotation commun (R).

7. Dispositif de retenue (17) selon l'une des revendications précédentes, **caractérisé en ce que**, le long de la couche d'obturation (15), les au moins trois éléments obturateurs (13) ont une forme triangulaire et/ou de segment de cercle.

8. Dispositif de retenue (17) selon l'une des revendications précédentes, **caractérisé en ce que** l'ouverture (12) destinée à recevoir l'instrument médical (33) du premier (1) et/ou du deuxième élément de réception (2) est conçue comme un trou et/ou une fente.

9. Dispositif de retenue selon l'une des revendications précédentes, **caractérisé en ce que** le premier et/ou le deuxième élément de réception est conçu en forme de roue.

10. Dispositif de retenue (17) selon la revendication 9, **caractérisé en ce que** le premier (1) et/ou le deuxième élément de réception (2) sont réalisés sous la forme d'une dentée et/ou d'une poulie.

11. Dispositif de retenue (17) selon l'une des revendications précédentes, comprenant en outre un élément d'entraînement motorisé (70) qui est conçu pour déplacer le premier élément de réception (1) par rapport au deuxième élément de réception (2) autour de l'axe de rotation commun (R).

12. Dispositif de retenue (17) selon l'une des revendications précédentes, comprenant en outre un élément d'immobilisation qui est conçu pour bloquer le premier élément de réception (1) par rapport au deuxième élément de réception (2).

13. Dispositif de retenue (17) selon l'une des revendications précédentes, comprenant en outre un élément d'immobilisation d'obturateur (75) qui est conçu pour bloquer les au moins trois éléments obturateurs (13).

14. Dispositif de retenue (17) selon l'une des revendications précédentes, le premier élément de réception (1) comprenant pour chacun des au moins trois éléments obturateurs (13) un deuxième élément d'accouplement (11) qui est évidé sous la forme d'un guide allongé et qui s'étend de manière rectiligne,
le deuxième élément de réception (2) comprenant pour chacun des au moins trois éléments obturateurs (13) un deuxième élément d'accouplement (11) qui est évidé sous la forme d'un guide allongé et qui s'étend de manière incurvée,
le premier (1) et le deuxième élément de réception (2) étant conçus en forme de roue,
le premier (1) et/ou le deuxième élément de réception (2) étant conçus sous la forme d'une roue dentée,
l'ouverture (12) destinée à recevoir l'instrument médical (33) du premier (1) et du deuxième élément de réception (2) étant conçue comme une fente et étant délimitée par l'axe de rotation commun (R),
les au moins trois éléments obturateurs (13) ayant une forme triangulaire, chacun des au moins trois éléments obturateurs (13) comportant deux premiers éléments d'accouplement (14) qui sont conçus comme des surélévations en forme de tige,
un premier des deux premiers éléments d'accouplement (14) de chacun des au moins trois éléments obturateurs (13) étant disposé sur un côté de l'élément obturateur (13) qui est dirigé vers le premier élément de réception (1),
un deuxième des deux premiers éléments d'accouplement (14) de chacun des au moins trois éléments obturateurs (13) étant disposé sur un côté de l'élément obturateur (13) qui est dirigé vers le deuxième élément de réception (2),
les deux premiers éléments d'accouplement (14) de chacun des au moins trois éléments obturateurs (13) ne venant pas en appui sur un axe de l'espace qui est parallèle à l'axe de rotation commun (R).
